Europäisches Patentamt

European Patent Office

Office européen des brevets

(19)

(11) Publication number: **0 305 210 B1**

(12) **EUROPEAN PATENT SPECIFICATION**

(45) Date of publication of patent specification: **08.12.93**

(51) Int. Cl.⁵: **G01N 1/28**, G01N 35/00, B01L 3/00

(21) Application number: **88307946.9**

(22) Date of filing: **26.08.88**

(54) **Apparatus and method for dilution and mixing of liquid samples.**

(30) Priority: **27.08.87 US 90026**
**05.11.87 US 117791**

(43) Date of publication of application:
**01.03.89 Bulletin 89/09**

(45) Publication of the grant of the patent:
**08.12.93 Bulletin 93/49**

(84) Designated Contracting States:
**AT BE CH DE ES FR GB GR IT LI LU NL SE**

(56) References cited:
**EP-A- 0 057 110**
**GB-A- 1 163 281**
**US-A- 3 799 742**
**US-A- 3 869 068**

(73) Proprietor: **Biotrack, Inc.**
**1058 Huff Avenue**
**Mountain View California 94043(US)**

(72) Inventor: **Gibbons, Ian**
**1003 Fremont Street**
**Menlo Park California 94025(US)**
Inventor: **Hillman, Robert S.**
**22774 Majestic Oak**
**Cupertino California 95019(US)**
Inventor: **Robertson, Channing R.**
**1089 Vernier Place**
**Stanford California 94305(US)**
Inventor: **Gorin, Michael M.**
**501 Forest Avenue No. 1010**
**Palo Alto California 94301(US)**
Inventor: **Cobb, Michael E.**
**967-1 Belmont Terrace**
**Sunnyvale California 94086(US)**

(74) Representative: **Harrison, David Christopher et al**
**MEWBURN ELLIS**
**2 Cursitor Street**
**London EC4A 1BO (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services
(3.10/3.6/3.3.1)

EP 0 305 210 B1

## Description

Technical Field

This invention relates to methods and apparatuses used for diluting and mixing liquids, particularly the automatic measuring and diluting of small volumes of liquids.

Background

There has been a recent period of explosive growth in the field of clinical analyses intended to be carried out by unskilled users. Numerous approaches have been developed which allow an unskilled user, such as a diabetic patient, to determine the presence and/or amount of an analyte in a sample, such as glucose in urine. The devices that carry out such analyses are generally intended to be "user friendly" in that they require little training and are essentially fool-proof in use. Typical of these devices are the so-called "dip-sticks". These devices are plastic strips with a reagent-containing matrix layered thereon. Sample is applied to the strip, and the presence or absence of an analyte is indicated by a color-forming reaction.

While such devices have proven useful for the qualitative determination of numerous substances in biological samples, not all analyses can be carried out in this manner. For example, some techniques require dilution and/or mixing of small quantities of sample. Measurement of extremely small amounts (e.g., microliter amounts) of liquid and the dilution thereof typically require significant training or the use of expensive equipment to carry out the dilution. Neither of these alternatives is convenient or easy to carry out.

Measuring and dilution of small samples of liquid is readily carried out in a number of automatic analyzers. However, these are not suitable for use in the home or in a doctor's office because of their size and expense. For example, many devices are available in which a sample of liquid is drawn into a conduit which is in the form of a capillary tube that acts as a metering device. However, this metering device is part of a large apparatus containing pistons and numerous other moving parts, such as vacuum pumps, that are required for movement of the sample and diluent. The precision with which such moving parts must be manufactured in order to retain liquid-tight seals significantly increase the cost of the device.

As an alternative to large automatic analyzers, small hand-held micropipets, such as the well known Eppendorf[R] pipet, have been devised. These pipets utilize a precision piston to draw sample or diluent into a small disposable tip. However, skill is required in the use of the pipet, and a number of precise manual operations must be carried out to successfully measure sample and diluent. Skill is also required in mixing the resulting small-volume solution.

Another technique that has been developed for the home uses a capillary tube to measure a sample of fluid. The entire capillary tube is then placed into a large container which holds a measured quantity of diluent or to which a measured quantity of diluent is added. However, such devices are not generally satisfactory in the hands of an unskilled user, since capillary tubes are easily broken and since contamination of the outside of the capillary results in volume error.

Accordingly, there is a need for simple and accurate methods and devices for measuring, diluting, mixing, and analyzing small quantities of sample.

Relevant Literature

West German published patent application DE3328964C1, publication date February 14, 1985, describes a device for the automatic, discontinuous sampling of fluids using a capillary tube that acts as a measuring device and which can be either dipped into a fluid being sampled or alternatively moved into a position from which the sample is transported with a diluent to an analyzer by a pump or suction. U.S. Patent No. 4,454,235 describes a capillary tube holder for liquid transfer in immunoassays. U.S. Patent No. 4,233,029 describes a liquid transport device formed by opposed surfaces spaced apart a distance effective to provide capillary flow of liquid without providing any means to control the rate of capillary flow. U.S. Patent Nos. 4,618,476 and 4,233,029 describe a similar capillary transport device having speed and meniscus control means. U.S. Patent No. 4,426,451 describes another similar capillary transport device including means for stopping flow between two zones, flow being resumed by the application of an externally-generated pressure. U.S. Patent Nos. 3,811,326; 3,992,150; 4,537,747; and 4,596,780 describe various processes and devices in which a capillary tube is used to take up a predetermined volume of the test solution and the charged capillary is in place in a cuvette or other container of liquid that is used as reagent or diluent. U.S. Patent No. 3,799,742 describes an apparatus in which a change in surface character from hydrophilic to hydrophobic is used to stop flow of a small sample, thereby metering the sample present.

## SUMMARY OF THE INVENTION

The present invention provides a self-contained dilution apparatus that does not require the use of externally generated force (except gravity) to move liquids between its various parts and provide for reproducible dilution of samples. In a first aspect, the invention provides an apparatus as set out in claim 1. Briefly, the apparatus comprises a fixed volume measuring chamber; a fixed volume receiving chamber in fluid receiving relationship to the measuring chamber; a gas vent in the receiving chamber; a stop flow junction between the measuring chamber and the receiving chamber; a sample application site in fluid donating relationship to the measuring chamber, wherein the vertical height difference between the sample application site and the stop flow junction is insufficient to provide flow through the stop flow junction when sample is applied to the sample site; and a diluent application site in fluid donating relationship to the measuring chamber. Means for starting flow at the stop flow junction are provided in some cases internally in the apparatus and in other cases are provided by external forces and/or devices. The stop flow junction uses backpressure caused by surface tension to stop flow of liquid under some circumstances while allowing flow under others. The stop flow junction acts as a valve but has no moving parts, relying on surface tension and the geometry of the junction to accomplish its function. Various means for starting flow include locating the diluent application site sufficiently above the stop flow junction to provide enough hydrostatic pressure to overcome the backpressure at the stop flow junction, including a movable arm or other device proximate to the stop flow junction in order to break surface tension, or providing a vibrator (optionally located externally to the apparatus) to break surface tension. In all cases in which external force is applied as a means for starting flow, this external force is not required for continued flow once flow resumes.

In operation, this embodiment of the apparatus is used in a method in which a sample is applied to the sample application site, whereby the liquid sample flows by capillary action or under the influence of gravity into the fixed volume measuring chamber. Flow stops when the sample reaches the stop flow junction. Diluent is then added to the diluent application site. Any necessary external force required to activate the means for starting flow is then applied, if necessary, so that diluent displaces the sample from the fixed volume measuring chamber into the fixed volume receiving chamber. The geometry of the measuring chamber is such that diluent displaces the sample rather than flowing through the sample without displacing it. Once the backpressure of the stop flow junction is overcome, no external force (other than gravity in some cases) is required for this liquid movement. A gas vent is provided in the receiving chamber in order to allow gas to escape and liquid to flow into the receiving chamber. Diluent continues to flow until the fixed volume receiving chamber is completely filled with a known mixture of sample and diluent.

Optional means for mixing can be provided in the receiving chamber. The means for mixing and means for starting flow can be different or the same (for example, a stirring bar can be used both to break surface tension at the stop flow junction and to mix the sample and diluent in the receiving chamber). In various embodiments of the invention, the receiving chamber or other parts of the apparatus can contain reagents and the receiving chamber can be utilized to provide a suitable location for an optical or other type of measurement. In other embodiments, means can be provided for removing sample from the receiving chamber for further operations at other locations.

In one such further embodiment, the apparatus provides for serial dilutions; i.e, dilution of a sample with a first diluent to obtain a mixture followed by dilution of the mixture with the same, a second, or a further diluent. At least one valve is required in this embodiment. The apparatus comprises a sample application site; a mixing chamber in fluid receiving relationship to the sample application site; a diluent application site in fluid donating relationship to the mixing chamber; a mixture isolating chamber hydrostatically connected to the mixing chamber; and first valve means selectively preventing flow from the diluent application site to the mixing chamber or second valve means selectively preventing flow between the mixing chamber and the mixture isolating chamber can be provided as part of the device or by external control of venting of a capillary track. The parts of the device are integrated into a cartridge in which the valves are preferably actuated by external solenoids, which can be preprogrammed. In use, the mixing chamber of the device is supplied with a predetermined volume of sample and a predetermined volume of a first liquid diluent, thereby providing a first mixture. The device itself can be used to meter these volumes, if desired, as described above. A valve controlling passage of liquid from the mixing chamber to the hydrostatically connected mixture measuring chamber is opened, whereby a hydrostatically determined portion of the first

3

EP 0 305 210 B1

mixture enters the mixture isolating chamber. The valve is then closed, isolating that portion of the first mixture from the remainder of the first mixture. This portion can then be transferred to a separate mixing chamber, or returned to the first mixing chamber, for dilution with a second diluent. Additional valves and chambers can be present if desired to provide for additional manipulation of the sample.

In a second aspect the present invention provides a method as set out in claim 12.

The present invention will be better understood by reference to the following detailed description of the invention when considered in conjuction with the attached drawings that form a part of the present specification, wherein:

Figure 1 is a vertical cross-section showing the internal liquid-contacting surfaces of a first embodiment of the invention.

Figure 2 is a vertical cross-section showing the internal liquid-contacting surfaces of a second embodiment of the invention.

Figure 3 is a vertical cross-section showing the internal liquid-contacting surfaces of a third embodiment of the invention.

Figure 4 is a vertical cross-section showing the internal liquid-contacting surfaces of a fourth embodiment of the invention.

Figure 5 is a vertical cross-section showing the internal liquid-contacting surfaces of a fifth embodiment of the invention.

Figure 6 is a vertical cross-section showing the internal liquid-contacting surfaces of a sixth embodiment of the invention.

Figure 7 is a horizontal cross-section showing the internal liquid-contacting surfaces of a seventh embodiment of the invention.

Figure 8 is a horizontal cross-section showing the internal liquid-contacting surfaces of an eighth embodiment of the invention.

Figure 9 is a vertical cross-section showing the internal liquid-contacting surfaces of a nineth embodiment of the invention.

Figure 10A and Figure 10B are horizontal and vertical cross-sections, respectively, showing the internal liquid-contacting surfaces of a tenth embodiment of the invention.

Figure 11 is a perspective view of an eleventh embodiment of the invention having a vertical sample measuring chamber.

Figure 12 is a perspective view of a twelfth embodiment of the invention having a horizontal sample measuring chamber.

Figure 13A provides a plan view and Figures 13B and 13C provide vertical cross-sectional views of a thirteenth embodiment of the invention.

Figure 14A provides a plan view and Figure 14B provides a vertical cross-sectional view of a fourteenth embodiment of the invention.

Figure 15A provides a plan view and Figure 15B provides a vertical cross-sectional view of a fifteenth embodiment of the invention.

Figure 16A is a perspective view and Figure 16B is a vertical cross-sectional view of a junction corresponding to junction 14 of Figure 13B modified to have a capillary channel to encourage flow of a sample from a capillary channel into a larger chamber.

Figures 17A and B are vertical cross-sections of a first multiple-dilution embodiment of the invention.

Figure 18 is a vertical cross-section of a second multiple-dilution embodiment of the invention in which an externally measured sample is added to the apparatus.

Figure 19 is a vertical cross-section of a third multiple-dilution embodiment of the invention.

Figure 20 is a vertical cross-section of a fourth multiple-dilution embodiment of the invention.

Figure 21 is a vertical cross-section of a fifth multiple-dilution embodiment of the invention.

Figure 22 is a plan view of three diluent application site covers for use with the embodiment of Figure 21.

Figure 23 is an expanded vertical cross-sectional view of a valve and the surrounding parts of the apparatus of Figure 17A.

Figure 24 is a perspective view of the valve of Figure 23.

Figure 25 is an expanded vertical cross-sectional view of a second embodiment of a valve of the apparatus of Figure 17A.

Figure 26 is a perspective view of the valve of Figure 25.

Figure 27 is a perspective view of a sixth multiple-dilution embodiment of the invention.

Figures 28A and B are vertical cross-sections of a seventh multiple-dilusion embodiment of the invention.

4

Figure 29 is a schematic diagram of reagents and their location when using a device of Figures 28A and B to carry out an analysis of hemoglobin Alc.

## DESCRIPTION OF SPECIFIC EMBODIMENTS

The present invention provides an apparatus and a method by which small samples can easily be measured and diluted. The apparatus is small, convenient to use, and requires no moving parts for the movement of fluid, gravity and capillary action being sufficient to provide all motive forces. Valves are provided to control the movement of fluid from chamber to chamber in some embodiments. The valves are integrated into the apparatus in most cases and, in preferred embodiments, are controlled by a simple push/release mechanism that can be controlled by an external solenoid. For some operations, valves can consist of externally controlled vent covers to control the flow of liquids in capillary spaces. Accordingly, the apparatus, referred to herein as a dilution and mixing cartridge, is easy to use, is inexpensive to manufacture, and can be used in a large number of procedures in which a dilution or a series of dilutions of a small sample is required. The cartridge is typically designed for insertion into a photometer or other device capable of making analytical readings directly on the diluted sample so that no further handling of the diluted sample is required.

The parts of the cartridge include a fixed volume measuring chamber, a fixed volume receiving (mixing) chamber in fluid receiving relationship to the measuring chamber, a vent to allow gas (e.g., air) to leave the receiving chamber, a stop flow junction between (preferably at the intersection of) the measuring chamber and the receiving chamber, a sample application site, and a diluent application site. Separate means for starting flow at the stop flow junction are provided in some embodiments. When a sample is present in the fixed volume measuring chamber but no diluent is present, the sample is prevented from flowing into the receiving chamber by backpressure created by surface tension at the stop flow junction.

The various parts and the function of the various parts can be understood by following the course of action as a sample is applied to the apparatus and is diluted. The following description follows this plan of organization.

The sample is a liquid and may be derived from any source, such as a physiological fluid; e.g., blood, saliva, occular lens fluid, cerebral spinal fluid, pus, sweat, exudate, urine, milk, or the like. The liquid sample may be subjected to prior treatment, such as preparing serum or plasma from blood or dissolving or suspending a solid in a liquid. Examples of sample treatments prior to application to the apparatus of the invention include concentration, filtration, distillation, dialysis, inactivation of natural components, chromatography, and addition of reagents. In addition to physiological fluids, other liquid samples can be employed. Examples of other liquid samples include process streams, water, plant fluids, chemical reaction media, biological growth media, and the like. For the most part, the liquid will be aqueous, although other liquids can be employed. Aqueous media may contain additional miscible liquids, particularly oxygenated organic solvents, such as lower alkanols, dimethyl formamide, dimethyl sulfoxide, acetone, and the like. Usually the solvents will be present in less than about 40 vol%, more usually in less than about 20 vol%, in order to maintain the high surface tension that is present in aqueous solutions. However, the apparatus of the invention can be modified as described below for use with liquids exhibiting different surface tensions.

The sample application site will generally be a cavity on a surface of the apparatus or may simply be an opening (optionally surrounded by a ring or tube) leading to the interior of the apparatus. The sample application site can contain a filter, for example, to separate red blood cells from plasma (see U.S. Application Serial No. 924,633, filed October 29, 1986), or may represent a connection between the apparatus of the invention and some other apparatus that manipulates the sample prior to its entering the present dilution apparatus. For example, the application site can be a recess into which a standard capillary tube will fit.

When the sample application site is a recess for insertion of a capillary tube, the capillary tube can act either as a convenient means for transferring the sample or can act as a measuring chamber, either by completely filling the capillary or by filling the capillary to a particular mark. The sample application site in such embodiments acts as a point of transfer.

In other cases, the sample application site will be a measuring chamber, such as a recess on an upper surface of the device into which sample is inserted. The application site can be provided with a raised lip surrounded by a catch basin so that the application site can be filled to overflowing, with excess sample overflowing into the catch basin. A defined volume of sample can therefore be readily obtained.

In still other cases, the sample application site can be a chamber having two channels leading away from the chamber. The first channel is or leads to an internal sample measuring chamber as described herein. The second channel is a drain that leads to an excess sample chamber. The excess sample channel is smaller than the measuring channel or is otherwise provided with means to restrict flow through the excess sample channel so that sample applied to the sample application site will flow primarily into the measuring chamber until the measuring chamber is filled, after which excess sample is drained away by the excess sample channel.

When sample is applied to the sample application site and the apparatus contains an internal sample measuring chamber, the liquid sample flows without the application of external force (except unassisted gravity) into the sample measuring chamber, which has a fixed volume. A capillary channel or non-capillary channel capable of transporting fluid can connect the sample application site to the measuring chamber, or the capillary or other channel exiting the sample application site can itself be the measuring chamber. The measuring chamber can be a capillary channel or chamber, in which case capillary action will aid or in some cases provide all the force necessary for filling the measuring chamber with sample from the sample application site. Capillary channels and chambers will generally have at least one dimension perpendicular to the flowpath in the range 0.01 to 2.0 mm, more generally 0.1 to 1.0 mm. However, larger measuring chambers are also possible. The sample measuring site is said to be in "fluid receiving relationship" to the sample measuring chamber in order to indicate that unassisted flow occurs.

The geometry of the measuring chamber is such that when diluent is added to the apparatus at a later step, essentially all of the sample in the measuring chamber will be expelled or drawn into the mixing chamber. One means of accomplishing this is by providing for smooth flow of diluent through the measuring chamber. A straight tube open at both ends is thus a preferred embodiment for this type of measuring chamber. In preferred embodiments of this type, diluent enters the measuring chamber in a front across the entire cross-sectional area of flow. This helps avoid mixing of diluent with sample and passage of diluent through the measuring chamber without expelling essentially all of the sample, which can occur if a small stream of diluent enters into a broader cross-sectional area of the measuring chamber.

In an alternate embodiment, the sample measuring chamber can terminate at a junction leading into a passageway between the diluent application site and the mixing chamber, both of which are later described. Passage of moving diluent past the junction will serve to draw sample into the mixing chamber. The passageway can be narrowed at the location of the junction to assist in drawing sample into the passageway and thus into the mixing chamber. This embodiment is particularly useful when the sample measuring chamber is a simple tube connecting the sample application site to the passageway between the diluent application site and the mixing chamber.

When sample flows into the fixed volume measuring chamber, flow stops when sample reaches the stop flow junction, so called because it marks the junction between the early part of the fluid track in which sample flows freely and the later part of the fluid track into which sample does not normally flow until the user has had time to initiate the dilution process. Since the stop flow junction exists at the limit of the flow path of the sample, it will be found at one end of the measuring chamber. In some cases, this same location will be the beginning of the receiving chamber (i.e., when the two chambers are directly connected). However, in other cases an additional channel may connect the stop flow junction to the receiving chamber.

It should be recognized that flow stop can occur both stably and metastably. A metastable flow stop is one in which flow stops on the macroscopic level but may resume without apparent cause after a time interval of a few seconds to a few minutes. Gradual creep of liquids along container walls or through microscopic or submicroscopic channels resulting from imperfections in the manufacturing process is believed to be the mechanism by which flow starts again once it has stopped. Additionally, small, undetectable vibrations (such as might be caused by persons walking near the apparatus or starting and stopping of nearby equipment, such as air-conditioning units) may also be sufficient to start flow in a metastable situation. However, there is no requirement of absolute stability since the apparatus is designed for addition of a diluent and eventual starting of flow at the stop flow junction. Accordingly, any flow stop which can be sustained for at least 10 seconds, preferably at least one minute, and more preferably at least five minutes, is sufficient for the purposes of this invention.

The stop flow junction is not a traditional valve as it has no moving parts. Rather, this junction relies on backpressure from the surface tension of the liquid sample to stop flow. This backpressure can be created in a number of ways. For example, backpressure is created when the cross-sectional area of the flow path increases in a region in which there is contact between the liquid and the container walls (e.g., when a small tube enters a larger chamber or when the cross-sectional area of a channel increases). Greater backpressure and more consistent operation is achieved when the increase in cross-sectional area of the flow path is abrupt rather than gradual, particularly when there is a break in capillarity in the sample flow path. Imperfections in the container walls during gradual widening of chambers may cause liquid to "creep" more on one side than another, thereby avoiding the creation of backpressure. Liquid can also creep around corners when imperfections are present. Unbalanced forces will also be present when the junction is not horizontal. A horizontal junction, for example, occurs when a vertical tube enters the top horizontal surface of a chamber. If a horizontal tube enters a vertical wall of a container, a vertical junction is present, and the pressure at the bottom of the stop flow junction will be greater than the pressure at the top of the junction, due to hydrostatic pressure caused by the different heights of liquid. Nonetheless, non-horizontal stop flow junctions can be created by reducing the diameter of the smaller channel containing liquid as it enters the larger area, thereby reducing the difference in pressure between the upper and lower portions of the junction.

In many cases, the junction will be formed when a small-diameter measuring tube (i.e., measuring chamber) enters a larger receiving chamber. A small measuring chamber can enter the larger receiving chamber at a right angle or at an angle other than a right angle. The angle between the internal wall of the small tube and the surface of the chamber in the latter case will be different at different locations around the circumference of the junction.

U.S. Patent No. 4,426,451, which is herein incorporated by reference, describes a number of stop flow junctions that it refers to as "meniscus control means" for use in a device in which there is capillary flow from one zone to another. The stop flow junctions described in that patent can be used in the apparatus of the present invention. However, the patent is not directed to stopping flow when the second zone is not a capillary zone. In contrast to the specific teachings of the patent, which indicate that the walls of the capillary chamber must gradually narrow and gradually expand in order to provide for flow stop, an abrupt widening has been found to be more effective in the practice of the present invention when the second chamber (here the receiving chamber) is not a capillary space. Although it is recognized that imperfections will exist on the molecular level, it is preferred that the junction be as sharp as possible from a macroscopic view point, approaching as closely as possible the ideal junction formed by the intersection of the plane (which can be curved) forming the walls of the measuring chamber with the plane forming the wall of the receiving chamber surface in which the stop flow junction is found. Maintaining a horizontal junction to avoid pressure differentials, reducing the area of the junction, changing the surface of the capillary so as to decrease the hydrophilic character (for aqueous solutions), providing smooth surfaces (rough surfaces encourage creep of liquid along the surface), and providing an abrupt change in cross-sectional area (preferably providing an angle between intersecting surfaces of about 90° or lower) all operate to prevent creep of liquid from one chamber to the other.

In general, for small (capillary size) junctions, the backpressure will be controlled by the smallest radius of curvature assumed by the meniscus. For example, when a capillary tube with a circular cross-section enters a larger space so that liquid bulges out into the space under hydrostatic pressure, the meniscus will be approximately spherical, and the backpressure ($\Delta p$) is given by the Young-Laplace equation: $\Delta p = 2\gamma/R$, where $\gamma$ is the surface tension of the sample fluid and $R$ is the radius of curvature. See, Miller and Neogi, "Interfacial Phenomena: Equilibrium and Dynamic Effects", Marcel Dekker, Inc., New York, 1985, and Davies and Riedeal "Interfacial Phenomena", 2nd Ed., Academic Press, New York, 1963. If the fluid meets the surface at an angle greater than 0°, this backpressure will be reduced by a geometric term. The radius, $R$, will change (become smaller) as the hydrostatic pressure increases, so that the backpressure and hydrostatic pressure balance. As hydrostatic pressure increases, $R$ reaches a minimum value (maximum curvature determined by the geometry of the device and the contact angle. The corresponding backpressure defines the maximum hydrostatic pressure sustainable by the stop flow junction.

On the other hand, junctions between other chambers and capillaries through which flow is intended to be continuous can be specifically designed to encourage rather than discourage flow. In order to encourage flow, the opposite approach is taken from that indicated above for stopping flow (e.g., increasing rather than reducing the area of the junction or providing a gradual change in cross-sectional area). In a preferred example of such flow junctions, a capillary groove extending in the direction of fluid flow can encourage flow past a junction. An example of such a groove is provided later in a specific embodiment.

Backpressure is also created when the surface that the liquid contacts changes to decrease adhesion between the liquid and the container wall (for example, when an aqueous sample moves from a hydrophilic to a hydrophobic surface). The surface properties of the various interior surfaces of the device of the invention can and generally will be controlled by various physical and/or chemical treatments. For a discussion of controlling surface properties of similar devices, see U.S Patent No. 4,756,884, which is incorporated herein by reference. For example, plastic surfaces can be treated to increase their hydrophilicity. Either the whole apparatus or specific parts can be treated. Alternatively, different parts of the apparatus can be made of different plastics. For capillary flow, contact angles of 0-90° are sufficient, preferably 10-85° and most preferably 30-70°. In order to provide these contact angles for aqueous samples, the capillary surfaces will be hydrophilic. For non-aqueous liquids, a hydrophobic surface would be appropriate. By using a combination of container wall geometry and surface wetability, a backpressure range of from 0 (no change in cross-sectional area or surface adhesion) to 20 cm $H_2O$ and higher can be achieved with water as the liquid. When the backpressure is 0, the location in question is not a stop flow junction. A stop flow junction occurs when there is sufficient backpressure to prevent the flow of sample past a particular point in the flow path; e.g., from the fixed volume measuring chamber to the fixed volume receiving chamber.

When the sample flow stops at the stop flow junction, the measuring chamber contains a fixed volume of sample. When diluent is added to the diluent application site and flow is restarted (see discussion below), diluent displaces the fixed volume of the sample into the receiving chamber and continues to flow into the receiving chamber in order to dilute the sample. The portion of the interior chambers that is displaced into the receiving chamber defines the measured, fixed volume. The fraction of the internal spaces of the apparatus that actually form the measuring chamber will depend upon the geometry of the apparatus but will be readily apparent from operation of the device.

Two ways by which flow can be started are to decrease the backpressue due to surface tension or to increase the hydrostatic pressure at the stop flow junction. In preferred embodiments of the invention, flow is started automatically when diluent is added by locating the diluent application site at a height sufficiently above the stop flow junction to provide increased forward hydrostatic pressure that is capable of overcoming the backpressure caused by surface tension. Use of gravity-created hydrostatic pressure allows sequential addition of sample and diluent (without requiring the use of external force on the apparatus) to both measure sample and measure and initially mix the diluent. Forward hydrostatic pressure can also be increased by re-orienting the device so the vertical height of the liquid column over the junction increases. Complete mixing of sample and diluent, of needed or desired, can be accomplished later in such devices by a magnetic stir bar or other means as described below.

Motion of the apparatus can also be used to start fluid flow. Either a single, sharp, short, start/stop movement or a vibrating motion is suitable. Neither a single sharp motion nor a vibration is itself capable of causing sustained fluid flow, since the starting and stopping motions cause forces to be exerted in opposite directions, and therefore would cause no net motion of the fluid when averaged out. However, an initial motion can cause a forward motion of the liquid sample at the stop flow junction so that the surface-tension/hydrostatic-pressure balance is overcome locally; the sample fluid meniscus then contacts a capillary region so that flow commences or a drop of liquid forms and falls into the receiving chamber.

Backpressure due to surface tension at the stop flow junction can be reduced by causing a contact to occur between sample at the junction and a movable part. For example, an apparatus can be prepared in which both sample and diluent can be added to the apparatus without starting flow at the resticted flow junction leading to the receiving chamber. A movable part can be included within the apparatus in order to break surface tension at the stop flow junction by contacting the sample at that location. It is possible, for example, to use a lever or any other movable part to restart flow. One embodiment would have a pin (on the tip of the lever) that actually touches the meniscus. Preferred embodiments of the invention using this technique for starting flow contain a mixing bar or similar device in the receiving chamber that is capable of contacting the liquid at the stop flow junction as well as mixing sample and diluent. Numerous magnetically operated stirring bars (not all of which are in the shape of bars, although they are generally referred to by this term because of the common bar shape) are known. These stirring bars typically comprise a magnetic or magnetically susceptible material embedded in a polytetrafluoroethylene or other inert matrix and are actuated by a moving magnetic field that is generated either mechanically (e.g., by rotating or otherwise moving a magnet attached to a motor) or electrically (e.g., by generating a rotating magnetic field, such as that which is used to turn the rotor of an electric motor, or a reciprocating electric current). By using a stirring bar sized closely to fit within the receiving chamber and placing the stop flow junction proximate to the bar location, the normal movement of the stirring bar can be utilized to contact any protrusion of sample meniscus at the stop flow junction. Such protrusions can exist because of hydrostatic pressure transmitted through liquid in the measuring chamber from sample and/or diluent present in the apparatus or because of the geometry of the junction. The bar can be held in place magnetically so that no contact is made during addition of sample to the apparatus. For example, a stirring bar in the shape of a bar can be rotated 90 degrees away from the angle at which contact would normally be made.

In addition to magnetic stirring bars, non-magnetic stirrers of various forms can be used. Mixing with such stirrers is accomplished by mechanical motion, during or after dilution is completed. For example, a sliding plate can be provided which moves back and forth in the receiving chamber when the apparatus is tilted from side to side.

If vibration is used to start flow, a number of variations are possible. For example, a vibrator may be made part of the apparatus. Alternatively, a vibrator may contact the apparatus externally at any location capable of transmitting the vibrational motion to the stop flow junction. This can be at any point of the apparatus if the apparatus is prepared from a rigid material, which is commonly the case. It is also possible to use the motion of the magnetic stir bar to cause vibrations without contact of the stir bar with liquid at the stop flow junction, since rotation of the stir bar will typically cause vibrations within the apparatus. If desired, the vibrations can be increased by including protrusions that the stirring bar strikes in the walls of the receiving chamber or by providing a rough lower surface on which the stirring bar rotates.

Depending on the location and geometry of the stop flow junction and receiving chamber, flow either continues automatically (by capillary action or hydrostatic pressure upon relief of a metastable condition) or additional motions (especially vibrations) or other actions (e.g., contacting the meniscus again) can be utilized to allow a dropwise flow of sample and diluent under the hydrostatic pressure of the diluent until the rising level of liquid in the receiving chamber contacts the region of the stop flow junction, at which time backpressure caused by surface tension is no longer possible and flow continues until the fixed volume receiving chamber is filled. There are no particular restraints on the geometry of the receiving chamber other than that smooth fluid flow be provided for in order to prevent trapping of gas bubbles. Providing entry of sample and diluent into a lower portion of the receiving chamber and providing an upper surface of the receiving chamber that slopes upward toward the vent both aid in avoiding trapped bubbles.

The vent can merely be a small hole terminated by a stop flow junction in order to avoid exit of liquid from the device or can be a more sophisticated vent designed for gas exit without exit of liquid (e.g., a microporous, hydrophobic plug capable of passing air but not hydrophilic liquids).

Although there is no theoretical upper limit on the size of samples that can be measured and diluted using an apparatus of the invention, the method and apparatus are particularly suitable for measuring and diluting small quantities of liquids. Accordingly, the measuring chamber will generally have a volume of from $0.1\mu L$ to $100\mu L$, preferably $1\mu L$ to $30\mu L$, and most preferably $3\mu L$ to $10\mu L$. The receiving chamber generally has a volume of from $3\mu L$ to $1000\mu L$, preferably $10\mu L$ to $300\mu L$, and most preferably $30\mu L$ to $100\mu L$, thereby providing dilution ratios of from $10^4$:1 to 3:1, preferably $10^3$:1 to 10:1, and most preferably 100:1 to 10:1. Channels through which capillary flow will take place will usually have opposing walls spaced in the range of about 0.01mm to 2mm, more usually about 0.1mm to 1mm. The capillary spaces can be tubular (which does not necessarily intend a circular cross-section but can be square or other regular shapes) or can represent the space formed by flat plates and side walls with the side walls being spaced further apart than a capillary distance. A tubular chamber with at least one flat side (e.g., a square cross-sectional area, a rectangle with adjacent sides differing in length by no more than a factor of 1:2 to 1:4, or a semicircular chamber) are preferred for ease of manufacture in cases where channels are being formed by the joining of two adjacent surfaces, one of which can be flat.

The variability of the sample volume and the diluent volume from device to device and sample to sample will be determined by a number of factors.

1. The geometry of the device, particularly the ratio of length to cross-section of the measuring chamber. In general, the greater the ratio the better the precision. This ratio will determine the extent to which the position of the sample fluid meniscus at flow stop relative to the stop flow junction affects variability.

2. Variation of the dimension of the surface capillary from device to device. State of the art suggests reproducability of about 1%.

3. Variations in the surface tension and contact angle of the surface from sample to sample. There is a limited range of possible values for typical samples of a given type; e.g., plasma. For the preferred dimensions, such variations are not expected to cause significant error.

4. Variability in the extent of displacement of sample by diluent - the factors here are: a) back flow of sample into the feeder tube; b) extent of mixing of diluent and sample in the measuring chamber. The variation from sample to sample will be controlled by variations in sample viscosity and density and diluent viscosity and density. The known variability in sample viscosity and density for many typical sample fluids (e.g, plasma) is not very great. It appears that the diluent viscosity and density should ideally not be very much different from that of the sample for best results.

Taking these factors into consideration, estimates of likely variation in sample volume using the preferred dimension range down to less than 1%. Similar considerations apply to the variability of diluent volume except that variation in sample properties become less important (due to dilution).

The time to fill the sample capillary can be calculated from well known physical principles (ref: Chemical Engineer's Handbook (1973) 5th Ed., Eds. R.H. Perry & C.H. Chilton, McGraw Hill, New York). In general the time will be minimal. Preferred fill times are less than 5 min; better is less than 1 min; ideally, less than 10 sec. After taking whatever measures are called for to cause the dilution (addition of diluent, activation of any mechanical or electrical device), the time which elapses prior to fluid flow may be significant. Desirable are times less than 1 min, preferably less than 10 sec, most preferably less than 1 sec. The delay in fluid flow apparently results from the creation of an initial metastable condition which is overcome with the passage of time, perhaps by the mechanisms described above in the discussion of an initial metastable flow stop.

The sample application site must be capable of containing sufficient sample to fill the measuring chamber and of allowing sufficiently rapid flow so that sample applied to the sample application site will flow directly to the measuring chamber without being lost by overflowing the sample application site.

The diluent application site must be capable of containing sufficient liquid diluent to fill the measuring chamber through which the diluent flows, any intervening or peripheral flow channels (such as diluent backflowing into the sample application site), and the remainder of the volume inside the receiving chamber not taken up by the sample (or by any movable part or stirring bar present in the receiving chamber). Since flow stops when the receiving chamber is filled with sample and diluent, the dilution ratio is determined by this last volume and the volume of the measuring chamber. For example, if the measuring chamber size is $5\mu l$ and the receiving chamber has a total interior volume of $120\mu l$ containing a stirring bar having a volume of $15\mu l$, the dilution ratio will be 20:1 (20 volumes of diluent, i.e., $100\mu l$, to each unit volume of sample, i.e., $5\mu l$).

When using the preferred small volumes in an apparatus of the invention, restricted-flow-junction backpressures in the range of from 0.1 to 20cm $H_2O$, preferably 0.3 to 10cm $H_2O$, and most preferably 1 to 5cm $H_2O$ are used when addition of diluent is intended to automatically restart flow. Backpressures greater than the respective upper limits set forth in the preceding sentence can be used when flow is to be started by some other means.

It should be recognized that statements in this specification indicating upper and lower limits of ranges are to be taken as individually designating a series of upper limits and a series of lower limits which can be utilized in any combination. For example, a typical upper limit and a preferred lower limit may be used in combination to define a range of intermediate preference.

In addition to providing sample dilution and mixing, an apparatus of the invention can also provide automatic incubation of the sample with a first reagent for a specified amount of time prior to mixing and incubating with a second reagent. The incubation times can be made independent of the filling operations. This is accomplished by providing reagents on different surfaces or areas of a surface of the receiving chamber. For example, a first reagent in suitable dry form can be present on the bottom surface of the receiving chamber and a second reagent on the top surface of the chamber. When sample first enters the receiving chamber, it will contact the first reagent. There will then be a time delay as liquid fills the receiving chamber during which an initial incubation can take place. Mixing can be provided during this time if desired. When the sample and diluent reach the upper surface of the receiving chamber, the second reagent is contacted and enters into the reaction. Any number of zones of reagent can be provided at different places in the receiving chamber. Incubation times can be controlled by varying the height of a band of reagent above the bottom surface of the receiving chamber (for chambers that fill from the bottom up) or otherwise locating the reagent at a point that is reached late in the filling process (e.g., vertical bands at different distances from the stop flow junction when the chamber fills horizontally), by varying the volume and shape of the receiving chamber, and by varying the rate at which diluent enters the reagent chamber.

This last variable can be controlled by providing a "flow restrictor" (which may be part of or different from the stop flow junction). For example, controlling the size of the opening between the diluent chamber and the measuring chamber will control flow between them. Additional factors which control the rate at which diluent enters the reagent chamber are the dimensions of the diluent chamber, its height above the stop flow junction, and the cross-sectional area of any point in the flow path through which diluent flows. Also, flow restriction can be achieved at the gas vent from the receiving chamber.

The time to fill the receiving chamber will be controlled by the construction parameters of the device and the viscosity and density of sample and diluent. The ranges of interest are 0.1 sec to 10 min; better is 1 sec to 2 min; best is 10 sec to 1 min. The design parameters described herein, which can be combined with simple experimentation, readily allow selection of a desired fill time.

By providing a diluent chamber much larger than the receiving chamber, variations in flow rates and therefore fill and incubation times will be minimized. However, some variation can still occur if a user does not completely fill the diluent chamber. Accordingly, more accurate control of dilution and incubation times can be achieved by providing a diluent chamber with an overflow chamber for excess diluent and instructing the user to fill the diluent chamber until overflow occurs, thereby providing the same diluent height during each operation of the device. Additional improvements can be achieved by providing a wide cross-sectional area for the diluent chamber so that variation in height from beginning to end of the filling operation is minimized. Such large chambers can be used if desired by filling to a line without providing for an overflow chamber. Sealed containers of diluent that is released by rupturing the container can also be used to provide control of fill and incubation times and to separated liquid reagents from solid reagents that may be present in the same apparatus.

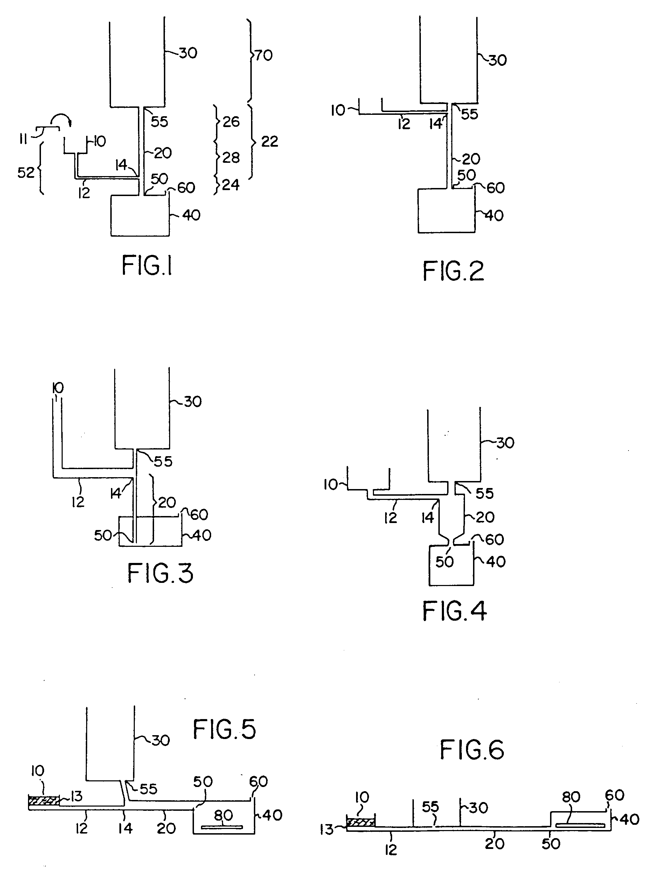

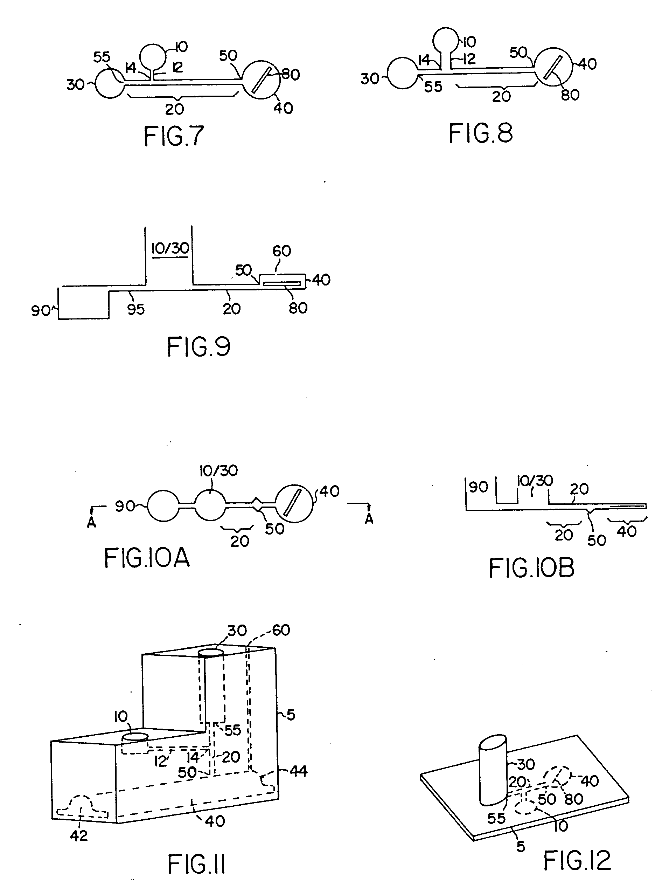

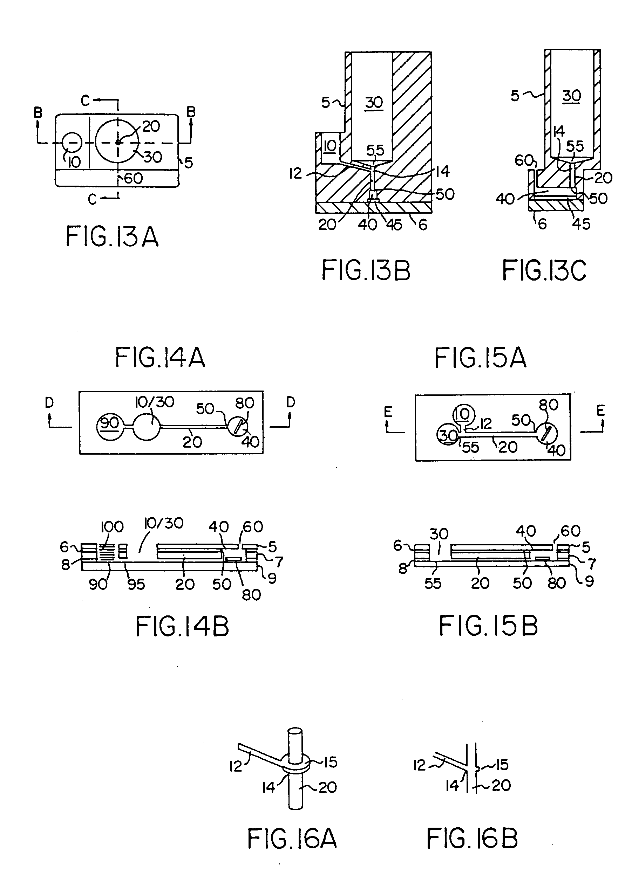

A first series of 16 figures is provided to illustrate a number of embodiments of the invention in which a single dilution takes place. The embodiments shown in the figures are not intended to be comprehensive, and numerous other embodiments within the scope of the appended claims will be apparent to those of ordinary skill in the field of the invention. In Figures 1-10, only the internal surfaces that contact liquid are shown. Figures 11 and 12 show entire apparatuses including both external surfaces (whose edges are marked by solid lines) and internal surfaces (whose edges are marked by dashed lines). Any of the embodiments shown by the internal surfaces of Figures 1-10 can be prepared in the form of an actual apparatus resembling Figure 11, Figure 12, or a combination or modification of these figures. For example, the embodiments shown schematically by means of internal liquid-contact surfaces in Figures 1 and 2 closely resemble the embodiment shown in its entirety in Figure 11. Figures 13-15 show embodiments of the invention in multiple views to show both external and internal surfaces.

Figure 1 is a schematic diagram showing a vertical cross-section of a first embodiment of the invention. Sample application site 10 is a shallow well or depression connected by a thin capillary 12 to measuring chamber 20 at junction 14. Measuring chamber 20 is vertical and has a section 22 above junction 14 and a section 24 below junction 14. When a liquid sample is added to application site 10, liquid flows through capillary 12 and into measuring chamber 20 at junction 14. In Figure 1 chamber 20 is also of capillary dimensions, by which is meant that the chamber is of sufficiently small cross-sectional area to be filled by capillary action. Capillary action is assisted by gravity in section 24 of chamber 20. When the sample reaches the bottom of chamber 20, flow stops at stop flow junction 50. The portion of chamber 20 above junction 14, identified as section 22 in Figure 1, is filled by capillary action. Hydrostatic pressure is also available for filling section 28 of chamber 20, but no hydrostatic pressure is available for filling section 26, which is above the fluid level in sample application site 10. In this embodiment of the invention, there is a stop flow junction 55 at the junction of measuring chamber 20 and diluent application site 30, which prevents excess sample from flowing into the diluent application site.

The geometry and surface characteristics of stop flow junction 50 are selected so as to provide sufficient backpressure to overcome the hydrostatic pressure of the sample. This hydrostatic pressure can be calculated from the maximum height of the sample above stop flow junction 50, which (in the embodiment shown in Figure 1) is the vertical height of the top of the sample application site (10) over stop flow junction 50 (i.e., height 52). The junction is formed by the intersection of the vertical tube that forms chamber 20 with the top horizontal surface of chamber 40. The principal design characteristic controlling backpressure is the cross-section of the junction, the area of which is decreased to provide higher backpressures and increased to produce lower ones.

When diluent is added to diluent application site 30, the resulting increase in hydrostatic pressure overcomes the backpressure at stop flow junction 50 and causes the sample in measuring chamber 20 to be driven by the diluent into receiving chamber 40. Air or any other gas contained in receiving chamber 40 is expelled through vent 60, which is too small to allow exit of fluid (i.e., the vent also acts as a stop flow junction, but one which has a backpressure that the hydrostatic pressure of the diluent is not able to overcome). Numerous other venting arrangements can also be utilized.

In this embodiment of the invention, the means for starting flow at the stop flow junction 50 is merely the additional height of liquid and resulting additional pressure head caused by locating diluent application site 30 higher than measuring chamber 20. Diluent application site 30 is sufficiently large when compared to receiving chamber 40 so that a sufficient pressure head is maintained throughout the dilution operation until receiving chamber 40 is filled (i.e., the pressure head is sufficient to overcome backpressure at stop flow junction 50 even when diluent application site 30 is partially depleted). The pressure head must also be sufficient to overcome any resistance to flow caused by viscous drag in the flow path.

A removable cap 11 (which may be completely removable or hinged or, more preferably, attached to sample application site 10 in a sliding arrangement) can be used to prevent flow of liquid back into the application site. If cover 11 is set in place prior to application of diluent, lack of a vent in this section of the apparatus will resist liquid flow back into the capillary 12. In the absense of a cap, when diluent is added to 30, some initial flow through capillaries 20 and 12 and back into sample application site 10 will occur. When sufficient diluent has been added to 30 so that backpressure at stop flow junction 50 is overcome, flow through measuring chamber 20 into receiving chamber 40 will also occur. A finite time is required to break the surface tension forces at 50, so that flow into receiving chamber 40 lags behind the initial backflow into capillary 12.

The direction and magnitude of fluid flows in the various capillaries and chambers depends on the dimensions of the device, the viscosity and density of the fluids, and the heights of the various liquid columns. After diluent has been added but before flow starts through stop flow junction 50, there will be backflow of sample (and/or diluent) from 20 into 12 and 10. After flow has been initiated through 50, the flow of sample (and/or diluent) from 20 is distributed between 12 and 24 and is controlled by:

A. Pressure head: as pressure increases, flow increases.

B. Tube length: as length increases, flow decreases.

C. Tube cross-section: as cross-section increases, flow increases.

D. Viscosity: as viscosity increases, flow decreases.

The sample will be (a) displaced into receiving chamber 40, (b) displaced into capillary 12, and (c) mixed with diluent in tube 20. The precise disposition of the sample will be controlled by the various factors listed. However, for a given device (and copies made to the same dimensions), specifying these factors ensures that a reproducible quantity of sample will be displaced into receiving chamber 40. It is the reproducibility of sample dilution that is most important in the dilution apparatus, since the actual extent of dilution can readily be adjusted by varying the size of the receiving chamber without significantly affecting the initial measuring of sample and/or types of flow in earlier stages within the device.

Thus (A) by using cap 11 to block backflow through 12, all the flow will be into 40, or (B) by making cross-section 12 much less than cross-section 20, length 24 short in relation to length 12, and pressure head 52 small in relation to pressure head of diluent (70 and 22), backflow into 12 is minimized and can be made negligible relative to flow through 24 into 40.

The precision of the volume of sample displaced into 40 may be compromised by mixing of sample and diluent in tube 20. If the extent of mixing in section 22 of capillary 20 varies from assay to assay, the composition of the fluid moving down section 24 into 40 will also vary. By minimizing the length of section 22, this problem can be made negligible. Other techniques for minimizing mixing have already been discussed.

Figure 2 shows a similar embodiment, which differs principally in that capillary 12 enters measuring chamber 20 at junction 14 much closer to diluent application site 30. This reduces the hydrostatic pressure at junction 14 and lessens the tendency of diluent and sample to flow backwards through capillary 12 into sample application site 10.

Figure 3 shows an additional variation of the embodiment shown in Figure 1. The principal differences are that channel 12 is larger than channel 20 with the effect that measuring chamber 20 begins at junction 14 and extends to restricted flow site 50, which is located well within receiving chamber 40 near the bottom of the chamber. The remainder of the apparatus and its operation are as described above. In this embodiment, adding diluent at site 30 will cause flow through junction 14 and capillary 12 back to sample application site 10. In this embodiment, however, no harm is caused by this reverse flow since the measurement section begins at junction 14 and does not include the connection itself or any part of a tube or other chamber extending from this junction to the diluent application site 30. By not closing off sample application site 10 and by providing channel 12 with a large diameter, backflow is encouraged.

Additionally, by extending tube 20 so that stop flow junction 50 is near the bottom of receiving chamber 40, it is no longer necessary to maintain a large excess of diluent in diluent application site 30. After the surface tension forces at 50 have been overcome and a small volume of sample and diluent has entered receiving chamber 40, the surface of the liquid contacts the bottom of chamber 40 and precludes interruption of flow. Flow of diluent into chamber 40 therefore continues until receiving chamber 40 is filled, even if the hydrostatic pressure head falls below that required to overcome normal backpressure at 50, since the absence of an air gap at stop flow junction 50 prevents backpressure from developing.

The three embodiments discussed above all have capillary chambers as measuring chamber 20. The embodiment shown in Figure 4 has a measuring chamber 20 that is not capable of filling by capillary action. Stop flow junction 50 is created by having a small exit hole leading from chamber 20 to chamber 40. Since hydrostatic pressure is independent of the volume of fluid, the design of the stop flow junction is no different from those described above. In such embodiments, however, junction 14, the location at which sample enters the chamber, must be located at the top of chamber 20 or sample application site 10 must provide sufficient hydrostatic pressure to fill chamber 20, since capillary action is not available to draw sample upward. The remaining parts of this apparatus and its operation are as described above.

The embodiments above utilize vertical measuring chambers 20. Horizontal (or mixed vertical/horizontal) arrangements are also possible. The embodiment shown in Figure 5 utilizes a horizontal measuring chamber 20. Figure 5 also shows a number of other possible variations. For example, a filter 13 is present in sample application site 10. This filter can be, for example, a glass fiber filter capable of separating red blood cells from whole blood, so that a whole blood sample applied at application site 10 is filtered to give a plasma sample withdrawn by capillary 12. Capillary action in chamber 20 is again utilized to fill this chamber. Stop flow junction 50 prevents flow of sample into receiving chamber 40. A second stop flow junction 55 prevents sample from entering diluent site 30. By locating the diluent application site vertically above stop flow junction 50, hydrostatic pressure is again available to drive sample into receiving chamber 40. This embodiment also contains a stirring bar 80 or other means for mixing the sample and diluent in chamber 40.

By slightly rearranging the geometry of the embodiment shown in Figure 5, it is possible to use mixing bar 80 as the means for starting flow. By locating the stop flow junction 50 at the bottom of chamber, as shown in Figure 6, the sample meniscus at 50 can be contacted by stirring bar 80 when it rotates, thereby allowing rotation of the stir bar to actuate flow. Since a high pressure head is not required, diluent application site 30 can be located at a lower height, insufficient to overcome backpressure at stop flow junction 50. However, diluent site 30 must provide sufficient hydrostatic pressure to fill chamber 40 once flow starts.

A variation on the geometry of the embodiment shown in Figure 6 is set forth in Figure 7, which shows a plan view from above rather than a vertical cross-section. Sample application site 10 is connected to a capillary measuring chamber 20 by connection 12. Junction 14 can be located at any point along measuring chamber 20. The cross-sectional area of tube 12 is much smaller than that of tube 20. Diluent application site 30 is located at one end of chamber 20 (connected by stop flow junction 55), while receiving chamber 40 is located at the other end (connected by stop flow junction 50). A stir bar 80 is located in receiving chamber 40, which is vented (not shown). Diluent application site 30 is located above or contains walls which extend sufficiently above the height of receiving chamber 40 to allow diluent to drive sample into the receiving chamber. However, the height difference is not sufficient to overcome the backpressure at stop flow junction 50. Rather, this backpressure is relieved when the sample meniscus at 50 is contacted by stirring bar/means for starting flow 80.

It is also possible to operate this apparatus, or any of the apparatuses described above, by using vibration as the means for starting flow. Motion of the fluid at the stop flow junction is sufficient to overcome backpressure if the amplitude and frequency of the vibration are sufficient. Only a slight hydrostatic pressure is then required to maintain flow, or flow can be maintained by using a capillary receiving chamber, as discussed below.

Figure 8 shows a variation of the embodiment shown in Figure 7, in which back flow through junction 14 to sample application site 10 is permitted. The cross-sectional area of tube 20 is much narrower than that of tube 12. Measuring chamber 20 therefore is measured from junction 14 to stop flow junction 50, as was described above for Figure 3. The remaining parts and operation of this apparatus are as described in Figure 7.

Figure 9 shows an embodiment of the invention in which a single application site serves both for application of sample and application of diluent. The cross-sectional view shown in Figure 8 has an application site 10/30 to which sample is initially added. The capillary channel 20 serves as the measuring chamber. A sump 90 is utilized to draw off excess sample through connection 95. A vent (62) is provided in sump 90. An absorbant material, such as cotton, can be present in sump 90 to absorb liquids, if desired. By providing sump 90, excess sample is withdrawn so that essentially no sample remains in application site 10/30 when diluent is later added. The small quantity of sample which remains in 10/30 can be made negligible compared to the sample volume (20) by appropriate choice of dimensions. Sufficient excess diluent is added to cause the sample held in measuring chamber 20 to be driven into receiving chamber 40. By sizing sump 90 so that it is sufficient to retain excess sample but insufficient to contain the amount of diluent added to application site 10/30, the apparatus can be operated with the certainty of driving sample into the receiving chamber. The remaining parts of the apparatus and their use are as described above.

Embodiments of the invention can be designed so as to rely solely upon capillary action to move fluids between parts. Figs. 10A and 10B represent horizontal and vertical cross-sectional views of such an apparatus. The principal difference between this and the previous embodiment of Figure 9 is that receiving chamber 40 is capable of filling by capillary action. Stop flow junction 50 is a small but abrupt widening in a capillary tube. By utilizing capillary spaces throughout, extremely small volumes can be measured and mixed. Sample applied at site 10/30 will fill chamber 20, but will not initially flow past stop flow junction 50. Diluent may be added to application site 10/30 without causing flow if the hydrostatic pressure remains sufficiently low. However, once vibration or a sharp motion causes the sample to cross the gap at site 50, flow continues by capillary action into receiving chamber 40. A separate vibrator may be provided or chamber 40 may be provided with a rough bottom surface or other indentations that make contact with stir bar 80 when in operation in order to use this stir bar to vibrate the apparatus sufficiently to cause flow to start.

Figure 11 is a perspective view showing an apparatus of the invention (including exterior surfaces shown by dashed lines) in which the measuring chamber 20 is vertical and the means for starting flow is the difference in vertical height between diluent application site 30 and stop flow junction 50. Receiving

EP 0 305 210 B1

chamber 40 traverses the length of the block in which the various chambers are formed so as to provide two end windows, 42 and 44, which define an optical path for measurement. Vent 60 is shown as passing upward through block 5 to an exit at a height equivalent to the upper portion of diluent application site 30. Placing the actual vent exit at this height and maintaining a small internal vent volume by maintaining a small vent diameter minimize error caused by entry of liquid into the vent. Since the vent exit is above the height of diluent remaining in diluent application site 30 after receiving chamber 40 has filled, hydrostatic pressure is not available to cause leakage out of the vent. The apparatus of Figure 11 can be prepared from two or more separate pieces having internal chambers or surface depressions that form the indicated internal cavities when the pieces are placed together.

Figure 12 shows a perspective view of an embodiment of the invention in which measuring chamber 20 is horizontal, in a manner similar shown in Figures 5 and 6 (vertical cross-sectional views) and 7 and 8 (plan views). In this embodiment, all parts except for diluent application site 30 can be prepared in the form of a two-piece, thin, plastic card-like device of the type described in U.S. Patent Application Serial No. 880,793, filed July 1, 1986. The diluent application site 30 is prepared by attaching an upward-extending cylinder to the surface of the flat device at the appropriate location. In the embodiment shown, stir bar 80 acts as the means for starting flow in a manner similar to that described for the embodiment shown in Figure 6. However, it is also possible to start flow utilizing hydrostatic pressure from diluent in application site 30 in a manner similar to that shown in Figure 5. The other parts of the apparatus and their operation have been discussed previously.

Figures 13-15 are described in detail in the examples which follow.

Figure 16A shows a perspective view of a junction that is not intended to stop flow at a junction between two chambers but is rather intended to encourage flow past that intersection. A capillary-sized groove 15 is provided to encourage flow from capillary 12 into chamber 20. Sample flows from channel 12 into groove 15. As shown in the vertical cross-sectional view of Figure 16B, junction 14 between capillary 12 and chamber 20 would otherwise act as a stop flow junction. By providing capillary groove 15, sample is encouraged to creep past junction 14. Groove 15 is of capillary dimensions and is capable of capturing sample from channel 12 and drawing it past junction 14. Groove 15 does not necessarily extend completely around chamber 20 as shown in Figure 16 but need only provide a capillary connection between the intersecting chambers.

All of the apparatuses of the invention whether or not discussed above can be used in a common method, although there are some variations in means for carrying out individual steps. A sample is added to a sample application site of an apparatus in all cases. Sample flows without the application of external energy (i.e., no pump, vacuum, air pressure, or the like is utilized) from the sample application site into a fixed volume measuring chamber. The fixed volume measuring chamber is terminated by one or more stop flow junction which stops sample flow prior to addition of diluent. Diluent is then added to a diluent application site of the same apparatus. The two steps of adding sample and adding diluent are usually carried out in the order stated since both the diluent application site and the sample application site are connected to the measuring chamber. If diluent is added first, diluent rather than sample would be measured in the measuring chamber. However, certain embodiments can have diluent present in the apparatus before sample is added if provision is made for exclusion of the diluent from the measuring chamber until sample is present in the measuring chamber. For example, a collapsible diluent bag can be placed in the diluent application site prior to addition of sample. After sample is added to the device and fills the measuring chamber, the bag is ruptured. A barrier of rupturable impermeable material can also be used to prevent diluent from entering the measuring chamber prematurely. Simultaneous addition of sample and diluent coupled with different flow rates into the measuring chamber (sample flow being faster than diluent flow) can also achieve the same result. The essential characteristic is that the sample chamber fills with sample prior to filling with diluent. Whether this is achieved by mechanical valves, rupturable seals, order of sample and diluent application, or any other means, is immaterial to the practice of the invention.

The diluent added is capable of flowing through the measuring chamber and stop flow junction and into a fixed volume measuring chamber. However, this flow does not necessarily occur without external activation to start flow. In some instances, no external activation is required, since the additional hydrostatic pressure caused by adding diluent to the diluent application site can be sufficient to overcome the backpressure due to surface tension at the stop flow junction. In other cases, the hydrostatic pressure is insufficient, and some other means of starting flow must be used, as described above.

During the filling of the receiving chamber, air or any other fluid trapped therein is released through a vent. The vent is either sufficiently small to form a stop flow junction so that liquid is trapped in the receiving chamber or the vent exit is located at a level higher than that of diluent in the diluent application site so that hydrostatic pressure cannot force significant volume of liquid out of the receiving chamber.

14

The apparatuses of the invention are quite simple, both in construction and operation compared to other automatic measuring devices. Typically, no moving valves or other parts are present in an embodiment that carries out a single dilution other than (in some embodiments) a stirring bar or a movable part for contacting the liquid surface at the stop flow junction in order to reinitiate flow. This part may be magnetically movable and can further be utilized as the stirring bar to mix the diluent and the sample in the receiving chamber.

The stop flow junction of the present invention can be readily designed using the criteria set forth herein and known physical principles relating backpressure and liquid flow to changes in chamber diameters, surface tensions of liquids, pressure heads, and the like. Some exemplary calculations are set forth in the examples that follow.

The parts of the cartridge embodiment that is capable of serial dilutions of a sample include a sample application site, a mixing chamber, a diluent application site, a mixture isolating and measuring chamber, and at least one valve controlling passage of fluid from the mixing chamber to the mixture isolating and measuring chamber. A second valve controlling passage of fluid from the diluent application site to the mixing chamber can be present as part of the cartridge or can be present as part of an apparatus into which the cartridge fits which operates a vent in the cartridge to control flow of a liquid in a capillary pathway. In some embodiments a sample measuring chamber will also be present. In contrast to the valveless first embodiment discussed above, a variety of simple valves are provided for controlling passage of fluid between internal chambers of the device. Only one valve need be present in a single cartridge, although multiple valves can be present. These valves allow multiple use of the same chambers (e.g., serial dilutions a single mixing chamber) in contrast to the linear flow arrangement present in the previously described apparatus as well as multiple dilution and mixing operations in multiple mixing chambers.

The various parts and the function of the various parts can be understood by following the course of action as a sample is applied to the apparatus and is serially diluted. The following description follows this plan of organization.

The sample, sample application site, diluent application site, sample measuring chamber, and mixing chamber can be as described above for the first embodiment in which a single dilution takes place. However, the apparatus of the second embodiment will differ in that at least one valve is present, for example controlling exit of waste fluid from the mixing chamber or entry of a portion of the mixed sample and diluent to a hydrostatically connected measuring chamber that samples and measures a portion of the mixture prepared in the mixing chamber. Additionally, other valves can be present in the apparatus of the present invention, such as a valve controlling flow of diluent from the diluent application site.

In certain embodiments of the invention, the mixing chamber can be used to determine the volume of diluent by providing a mixing chamber smaller than the diluent application site. In other cases, volume of diluent is determined by the volume of the diluent application site, in which case the mixing chamber has a volume at least as great as and usually larger than the combined volume of sample and diluent.

There are no particular restraints on the geometry of the mixing chamber other than that smooth fluid flow be provided for in order to prevent trapping of gas bubbles. Providing entry of sample and diluent into a lower portion of the receiving chamber and providing an upper surface of the receiving chamber that slopes upward toward the vent both aid in avoiding trapped bubbles. If the mixing chamber is larger than the combined volume of sample and diluent, the vent is preferably in the non-wetted upper portion of the mixing chamber. Vents are described above in more detail. A vent or other means to allow exit of trapped air must be provided at every location in which the trapping of air would interfere with the passage of liquids between the various chambers and/or channels of the device.

Sizes of channels and chambers in the serial dilution cartridge embodiment will be similar to those in the single dilution embodiment.

Serial dilution and mixing capabilities are provided by a mixture measuring and isolating chamber hydrostatically connected to the mixing chamber, and a valve controlling passage of fluids from the mixing chamber to the mixture isolating chamber. The first dilution takes place as indicated above during which time the indicated valve is closed to prevent escape of liquid from the mixing chamber. After the first mixture is formed, the valve controlling flow to the mixture measuring chamber is opened and fluid flows from the mixing chamber under the influence of hydrostatic pressure and/or capillary attraction. The portion of the mixture isolating chamber into which the mixture flows is smaller in volume than the total volume of mixed sample and diluent. This volume is determined by the geometry of the chamber, the amount of hydrostatic pressure available from liquid in the mixing chamber, and any capillary forces that are present. Various geometries can be provided for the mixture isolating chamber depending on whether the intent is to carry out a second dilution in the original mixing chamber or to transport the isolated portion of the mixed sample and diluent to another location for further dilution and/or analysis. For example, the mixture isolating chamber can be a tube (which does not imply circular cross-section), at least a portion of which extends upwardly from the connecting point between the mixing chamber and the mixture measuring chamber. When the valve is open, the mixture flows into the mixture isolating chamber until the level of liquids in the two chambers becomes equal, thereby equalizing hydrostatic pressure (assuming that capillary action is negligible). After this portion of the mixture has been isolated by closing the valve, the remainder of the mixture can be drained from the mixing chamber by opening a second valve that leads to a waste fluid exit in the mixing chamber. After the second valve is closed, opening the first valve allows the isolated portion of the mixture to return to the mixing chamber. A second dilution can then take place in the mixing chamber.

Alternatively, a portion of the mixture measuring chamber can extend below the first valve, for example in a V or U shape, with another portion extending upward. Providing a third valve at the low point of the mixture measuring chamber allows the measured portion of the first mixture to be drained into a second mixing chamber. In this embodiment, there is no requirement for the waste fluid exit in the mixing chamber since there is no need to remove the remainder of the first diluted mixture from the first mixing chamber. In this embodiment, the second measurement can also resemble the first; i.e., a measuring chamber terminated by a stop flow junction can be used to measure the mixture for later dilution by a second dilutent.

In either embodiment, the diameter of the measuring chamber can be of capillary dimensions so that capillary force is significant in determining the level to which mixture rises in the mixture measuring chamber. This height can readily be regulated by providing a vent or large diameter segment (such as a bubble chamber) to break capillarity.

The apparatus of the invention can be designed for use with a particular assay or can be designed and prepared as an apparatus in which multiple assays can be carried out, depending on the order in which various valves are opended and closed and the contents of the various diluents, which can contain reagents for the development of a detectable signal (e.g., a color reaction) that depends on the presence of an analyte in the sample.

Any type of valve that will control the passage of liquids between chambers and/or channels can be used in the apparatus of the present invention. Simple valves that can be actuated to move between an open and a closed position by the application and release of a simple external force are preferred.

Examples of such valves include resilient blocking members that are present in or adjacent to a liquid flowpath. For example, a resilient blocking member can be present in a converging or diverging pathway so that the narrow portion of the pathway is blocked by the resilient blocking member when the blocking member is in its normal position. Application of force in a direction generally away from the restricted portion of the flowpath and toward the wider portion of the flowpath will open the valve by moving the blocking member away from the narrow walls of the flowpath. Alternatively, a normally open valve can be provided which is blocked by movement of a resilient blocking member to a location that cuts off flow of liquid. Specific examples of such valves are set forth in more detail below.

Other examples of such valves include sliding pins closely engaging a channel that laterally traverses a fluid flowpath. The pin has a segment capable of obstructing flow through the flowpath when the pin is in a first position and a segment capable of allowing flow through the flowpath when the pin is in a second position. Examples of such pins include rectangular pins having a flowpath channel between two opposite faces of the pin, the flowpath channel being out of register when the block is in a closed position and in register with the principal flowpath when the block valve is open. Pins with circular cross-sections can be used by providing an obstructing segment of the pin that snugly engages the channel in which the pin fits and obstructs the flowpath when the pin is in a closed position. A smaller cross-sectional area (such as is present in the handle of a dumbbell) provides an annular flowpath around the smaller, central portion of the pin when the pin valve is in the open position.

A resilient member can be provided to bias the pin into either the closed or the open position. A force acting on the pin can then slide the pin to a second location so that the pin valve is in the alternate position.

In preferred embodiments, access for the application of an external force on the pin is provided so that the pin can be moved between its two positions. For example, a section of the pin that protrudes externally from the apparatus can be provided so that a force acting parallel to the sliding axis of the pin can move the pin from its first biased position to a second position by acting against the direction of the biasing force. Alternatively, an aperture leading from a face of the pin opposite the biasing force to the external environment can be provided. Externally applied pressure, such as from compressed air or a finger of an external apparatus that enters the aperture, can be used to slide the pin between its open and closed positions. A resilient seal can be provided to prevent loss of liquid through the aperture while allowing force to be applied to the pin. Such seals can also be provided for the resilient blocking members described above.

The valves that can be used as integral parts of a cartridge of the present invention are not limited to those specifically exemplified here. Rather, any valve can be used that can control the flow of liquids through small flowpaths, such as flexible walls of a flowpath that can be compressed to restrict flow of liquid through the flowpath. Additionally, a dissolvable barrier can be provided in instances where an initially closed valve will be opened once and then maintained in the open position.

It is also possible to provide an external valve. For example, a flowpath through which capillary flow occurs can be blocked by closing an external vent. When the external vent is closed, liquid cannot enter the capillary pathway because of air or other gases in the capillary pathway. Opening the vent allows liquid to enter the capillary pathway. If the vent is closed while liquid is contained in the capillary pathway, the isolated liquid can later be used for other manipulations.

Valves consisting of external vent controls can be used in any situation where flow occurs through a capillary pathway (so that trapped air is effective to control flow of liquids) and where no liquid is stored in the cartridge prior to use. In many cases it is desirable to store premeasured diluents (which can contain reagents) in the cartridge when the cartridge is delivered to an end user. Internal mechanical valves are preferred for such uses in order to prevent accidental leakage. Alternatively, liquid diluents can be sealed in glass or other rupturable containers in the diluent application sites. An external finger can be used to rupture the container and provide diluent at the diluent application site.

By providing valves that can be operated by a simple externally applied force, a cartridge-like device can be provided in which the valves are opened and closed in a predetermined manner by an analytical device into which the cartridge is inserted. This analytical device can contain various optical and/or other types of sensors for detecting the presence of liquids or analytes in various mixing and/or measuring chambers of the cartridge in addition to providing means for opening and closing the valves.

Reagents can be provided at various locations in a device of the invention. Incubation times can be controlled by either manual operation of valves or by a mechanically or electronically stored program in the device into which the cartridge is inserted. The program would control the order and timing of opening and closing valves. The programmed device would contain solenoids or other means for providing force to open and/or close valves. In embodiments in which flow through a capillary pathway is being controlled by the opening and closing of a vent, a movable sealing pad that is capable of closing the vent will form part of the external programmed device into which the cartridge is inserted.

A second series of Figures is provided to illustrate a number of variations on this second embodiment of the invention. The embodiments shown in the Figures are not intended to be comprehensive, and numerous other embodiments within the scope of the appended claims will be apparent to those of ordinary skill in the field of the invention.

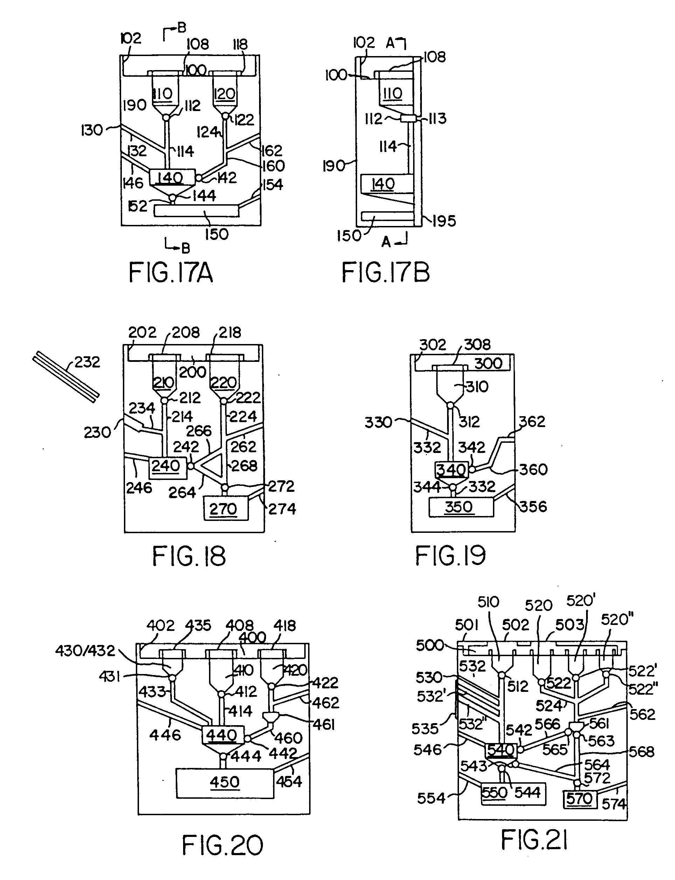

Figure 17A is a vertical cross-section of a first embodiment of the invention in which line A-A shows the location of the cross-sectional view in Figure 17B. Line A-A in Figure 17B, which is also a vertical cross-section of the first embodiment, shows the location of the view shown in Figure 17A. Sample application site 130 is located in a side face of body member 190. Diluent application site 110 is a cavity in an upper face of block 190. Valve 112 prevents premature passage of diluent from diluent application site 110 through passageway 114 to mixing chamber 140. Sample measuring chamber 132 connects sample application site 130 to fluid passageway 114. A stop flow junction is present at the intersection of sample measuring chamber 132 and passage 114. Vent 146 allows air in mixing chamber 140 to exit from the chamber when sample and diluent enter the chamber. Valves 142 and 144 prevent premature exit of mixture from mixing chamber 140. Valve 142 controls passage of liquid between mixing chamber 140 and isolating chamber 160, which is a narrow channel sloping upward from valve 142. Valve 144 allows excess mixture to pass from mixing chamber 140 through waste liquid channel 152 to waste liquid container 150, which is connected by vent 154 to the external environment. Vent 162 allows entry of liquid from mixing chamber 140 into isolating chamber 160 when vent 142 is open. Second diluent application site 120 is controlled by valve 122 which selectively prevents diluent from flowing through connecting channel 124 into isolating chamber 160 and through valve 142 back into mixing chamber 140. Body member 190 is provided with a lip 102 around the perimeter of the body member to provide a space 100 that acts as a catch basin for excess diluent. Diluent is measured by filling diluent application sites 110 and/or 120 and allowing a small amount of diluent to overflow lip 108 or 118, respectively, and flow into lower catch basin space 100, thereby ensuring complete filling of the diluent application site (and therefore accurate measurement of diluent). An access channel 113 is provided for application of external pressure to valve 112 and resulting movement of the valve between open and closed positions. Details of exemplary valves are set forth in later Figures.

The apparatus of Figure 17 can be used in the following manner, among others. A liquid sample, such as a drop of blood adhering to a finger after a finger stick, is touched to sample application site 130. A measured amount is drawn by capillary action into sample measuring chamber 132. Diluent is then added to diluent application site 110 until diluent overflows lip 108. Valve 112 is then opened to allow the measured amount of diluent in diluent application site 110 to flow through passage 114 into mixing chamber 140. As diluent flows past the stop flow junction at the junction of chamber 132 and passageway 114, the sample is drawn into the diluent. Valves 142 and 144 are both closed during this first mixing. Air that would otherwise be trapped in mixing chamber 140 exits through vent 146. Mixing in chamber 140 can be facilitated by including within the chamber a mixing bar or by rocking the complete apparatus back and forth.

Vent 142 is then opened to allow a hydrostatically controlled portion of the mixture to enter mixture isolation chamber 160. Mixture will rise into chamber 160 until hydrostatic and capillary forces are balanced. Valve 142 is then closed, isolating a portion of the mixture in chamber 160. The remainder of the mixture in chamber 140 is then drained through exit channel 152 into waste chamber 150 by opening valve 144. Valve 144 is closed and valve 142 is opened to allow reentry of the isolated mixture into mixing chamber 140. Diluent is then added to second diluent application site 120 and valve 122 is opened to allow dilution of the isolated portion of the mixture with the second diluent. By providing chambers of appropriate sizes, further dilution operations can be carried out by reisolating a portion of the mixture in chamber 160 followed by addition of a third or further diluent.

Apparatuses of the invention can readily be made by forming all cavities and passageways in body member 190 as shown in Figure 17B. A cover plate 195 is then used to form the internal cavities, with any access channels (such as access channel 113 shown for valve 112) or vents being provided in the cover panel as desired.

Figure 18 shows a second embodiment of the invention in which a separate capillary tube 232 is provided for obtaining and/or measuring a sample. Sample application site 230 in this embodiment is a recess into which capillary tube 232 fits. Connecting channel 234 allows sample to pass into passageway 214. Many of the features in this embodiment are strictly analogous to the features of the embodiment shown in Figure 17. Such features are identified by a reference number in which the last two digits of the reference number are identical to the last two digits of the reference number in Figure 17. The first digit of the reference number identifies the particular Figure. For example, valve 212 in Figure 18 is identical in function to valve 112 in Figure 17. Accordingly, the remainder of the description of this and other Figures will be principally directed to the differences between embodiments.

Mixing chamber 240 of Figure 18 differs from the mixing chamber of the embodiment shown in Figure 17 in that no waste liquid exit is provided. Instead, a single dilution takes place in chamber 240 after which valve 242 is opened. Mixture flows under hydrostatic control into descending arm 264 of the sample isolating chamber of this embodiment and then upward into ascending channel 266 and/or vertical channel 268. Valve 242 is then closed and a second mixing operation occurs in chamber 270. Valve 272 is opened to allow sample to flow into second mixing chamber 270 after which valve 222 is opened to allow diluent in diluent application 220 to enter chamber 270. Providing a three-part isolation chamber in the manner shown in Figure 18 prevents inadvertent retention of the first mixture in channel 264, which would be likely to occur if channel 266 were not present. Channel 266 provides access for air from vent 262 so that channel 264 can freely drain into second mixing chamber 270.

Figure 19 shows an embodiment in which serial dilutions can take place using a single diluent application site. Isolation chamber 360 is connected to the external environment by vent 362 but is not itself connected to a second diluent application site. After sample and diluent from diluent application site 310 have entered mixing chamber 340 to form a first mixture, valve 342 is sequentially opened and closed to isolate a hydrostatically determined portion of the first mixture in chamber 360. Valve 344 is then opened to drain the remainder of the first mixture into waste liquid chamber 350, after which valve 344 is closed. Valve 342 is then reopened to allow the isolated portion of the first mixture in chamber 360 to reenter chamber 340. At some time after the closing of valve 312, a second diluent (or a second volume of the first diluent) is added to diluent application site 310. Valve 312 is then reopened to allow formation of a second mixture in mixing chamber 340. This operation can be repeated as often as desired or until the capacity of waste chamber 350 is exhausted.

Figure 20 shows an embodiment of the invention in which the sample application site and sample measuring site are the same. Passage of sample from the sample application/measuring site 430/432 is controlled by valve 431. Sample passes through passageway 433 to mixing chamber 440 where it mixes with diluent from diluent application site 410. After mixing, valve 442 is sequentially opened and closed to isolate a portion of the first mixture in chamber 460. A bubble chamber (non-capillary space) 461 is provided to prevent capillarity from drawing excess liquid into chamber 460. The remaining parts of this embodiment and its mode of operation are as described for Figure 17.

Figure 21 shows a complex embodiment of the invention in which multiple analyses can be carried out, depending on the combination of sample application sites, diluent application sites, and valving operations. Multiple sample measuring chambers 532, 532′ and 532″ of different volumes are provided to allow for the measurement of different sizes of samples. Similarly, diluent application sites 520, 520′ and 520″ of different volumes can also be provided, each controlled respectively by valves 522, 522′ and 522″. Both a waste liquid chamber 550 and a second mixing chamber 570 are provided so that mixing (and subsequent measurements) can take place in either mixing chamber. Alternate isolating chambers are provided under the control of different valving systems. For example, once a first mixture has been formed in chamber 540, valve 542 can be opened while valve 543 remains closed. Channel 566 therefore acts independently of channels 564 and 568 to isolate a portion of the first mixture. Alternatively, valve 542 can remain closed while valves 543 and 563 are opened. Under these circumstances, channels 564 and 568 act as the mixture isolating chamber. After the remaining mixture in mixing chamber 540 is drained into waste liquid chamber 550, valves 543 and 563 are opened to allow the trapped portion of the first mixture to drain into second mixing chamber 570. Channel 566 is not needed in this sequence of steps, unlike the embodiment of Figure 18, since draining chamber 540 allows vent 546 to supply air for the mixture trapped in channel 564.

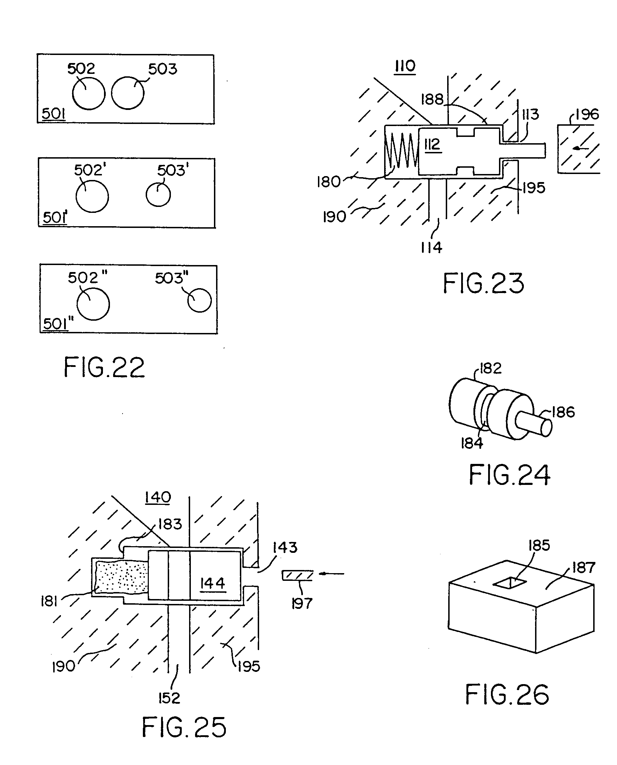

By providing a multiple use cartridge, such as that shown in Figure 21, different analyses can be carried out by selecting proper combinations of sample application sites and diluent application sites (to allow for different degrees of dilution) and by selecting diluents containing different reagents. In order to simplify use of the cartridge for a particular type of application, covers can be provided which allow access to only selected sample and/or diluent application sites. For example, Figure 21 shows a sample application site cover 535, which can be, for example, tape applied to cover all but one of the sample application sites for a particular assay. Likewise, cover 501 allows access through channels 502 and 503 only to diluent application sites 510 and 520. As shown in Figure 22, alternate covers can be provided for different analyses in which access channels are present in different locations to allow access to different diluent application sites.

Although numerous types of valves can be used in an apparatus of the invention, particularly preferred embodiments are shown in Figures 23-26. Figure 23 is a cross-sectional view of a valve such as might be present as valve 112 of the embodiment of Figure 17. A channel 188 is present in the apparatus, which here is formed from base member 190 and cover plate 195. This channel traverses a flowpath of sample from diluent application site 110 to passageway 114 (which leads to mixing chamber 140). The valve shown in cross-section in Figure 23 is shown in a perspective view in Figure 24. The portion of the valve that slides in the channel consists of a cylindrical pin with sections having different diameters. An obstructing segment 182, snugly fitting channel 188, obstructs the passage of fluid from chamber 110 to passageway 114 when the pin is in the position shown in Figure 23. The pin is maintained in this position by a resilient member 180, such as a spring, which presses outward on the body of valve 112. The depth of channel 188 in cover plate 195 is selected to retain pin 112 at the proper location. A projection 186 is provided on the end of pin 112 in order that an externally applied force operating parallel to and against the force produced by resilient member 180, which biases the valve into the closed position, can slide pin 112 in channel 188 to an open position in which segment 184 moves into the flowpath of liquid. Section 184 of pin 112 is of smaller diameter than segment 182 so that an annular flowpath of liquid is provided around the central portion of valve 112 at segment 184. For example, an actuating member 196, which is part of the apparatus into which the cartridge of the invention fits, can move in the direction shown by the arrow in Figure 23 to act on protruding portion 186 of pin 112. When actuating member 196 contacts cover plate 195, thereby moving pin 112 to its maximum extent, valve 112 is in the maximum open position.

A second embodiment of a valve is shown in Figures 25 and 26 with Figure 25 being a vertical cross-sectional view and Figure 26 being a perspective view of the sliding pin. In this case the pin is rectangular with a channel 185 between opposite faces to allow passage of liquid. A portion of the same face 187 obstructs passage of liquid when the pin moves to the closed position. The valve of Figure 25 differs from the valve of Figure 23 in several ways. Pin 144 is biased by a foam pad 181 to a normally open position rather than a normally closed position. Access channel 143 is provided in cover plate 195, similar to access channel 113 of Figure 23. However, no projection is provided on the face of pin 144. Instead, a pin 197 is provided as part of the analytical apparatus into which the cartridge fits. Pin 197 is located so as to enter channel 143 when moved in the direction shown by the arrow in Figure 25. This embodiment provides easily actuated valves but avoids projections on the outer surface of the cartridge which might accidently be triggered by handling. Channel 188 has shoulders 183 which prevent excessive movement of pin 144.

As indicated by the numbering system used, valve 144 of Figure 25 is equivalent to valve 144 of Figure 17. However, the valves of Figures 23 and 25 could be used at any location in the apparatus of the invention in which a valve is called for. In addition, many variations of valves of this type will be apparent to those skilled in the art from the description of the specific valves.

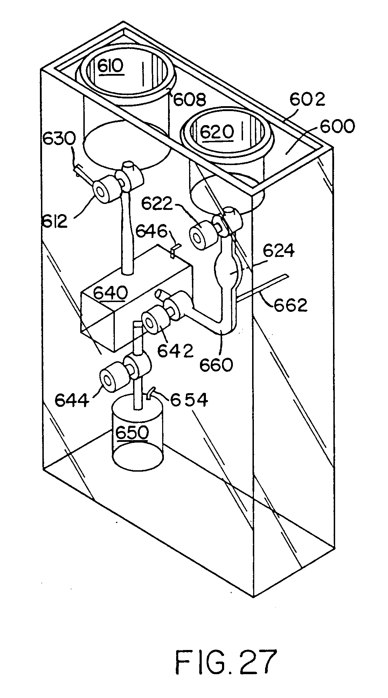

The Figures described above are not drawn to scale but are intended to indicate relative location and operation of some of the many possible variations of an apparatus of the invention. Figure 27 is a perspective drawing in scale of an apparatus of the invention that resembles the device shown schematically in Figures 17A and 17B. The last two digits of the reference numbers correspond with the last two digits of the reference numbers in Figures 17A and 17B. Valves 612, 622, 642, and 644 are of the type shown in Figures 23 and 24 except that projection 186 of the pin shown in Figure 24 is not present. Instead, the pin is contacted internally as shown in Figure 25.

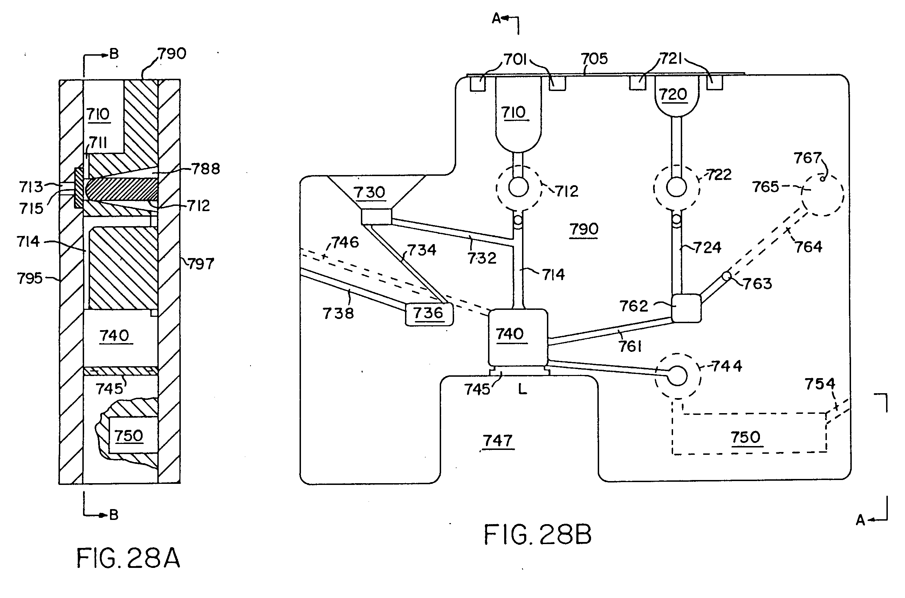

Figure 28A is a vertical cross-section of a seventh embodiment of the invention in which line B-B shows the location of the cross-sectional view in Figure 28B. Line A-A in Figure 28B, which is also a vertical cross-section of the seventh embodiment, shows the location of the view shown in Figure 28A. The device as shown is prepared from a base piece 790 and two cover plates 795 and 797. Most of the passageways and chambers are molded into base piece 790. Cover plate 795 contains apertures, such as aperture 713, through which force can be applied to operate internal valves, such as valve 712. In this embodiment, a resilient blocking member 712 is present in a channel 788 that diverges in the direction of the flowpath. The narrow end of channel 788 is blocked by one end of resilient blocking member 712, which is held in the blocking position by internal compression forces exerted when cover plate 797 traps blocking member 712 in channel 788. A resilient seal 715 is provided so that force can be applied through aperture 713 to blocking member 712 without leakage of sample or diluent from the device. Pressure on the end of resilient member 712 pushes it away from the narrow blocking portion of channel 788 toward the wider portion where flow can occur. In an actual device, resilient blocking member 712 could be approximately the size and shape of a thin lead pencil eraser and could be made of silicon rubber.

When valve 712 is open, diluent flows from chamber 710 through channel 711 along the front face of block 790 and then through 788 containing resilient blocking member (valve) 712. The flowpath continues into fluid passageway 714 at the rear of block 790 where liquid enters a channel traversing block 790 and then continuing across the front face of block 790 until liquid enters mixing chamber 740. Access panel 745 is provided to chamber 740 so that reagents can be added to the chamber during the manufacturing process, if desired.

Sample application site 730 is provided in an upper face of block 790. Two channels lead away from the sample application site. One channel is measuring chamber 732, which operates as described above for similar measuring chambers. An additional channel (734) is provided to remove excess sample from sample application site 730. Channel 734 is smaller than channel 732 so that sample initially flows preferentially into measuring channel 732. Excess sample channel 734 then leads excess sample into excess sample chamber 736, which is vented through vent 738 to the atmosphere. A measured volume of sample is therefore contained in channel 732 regardless of the amount of sample applied to application site 730.

Recess 747 is provided in a lower surface of block 790 immediately below chamber 740 in which mixing occurs. This recess allows close approach of a magnet or other means to activate a stirring bar or plate retained in chamber 740 while still allowing waste liquid chamber 750 to be located below (although displaced from) mixing chamber 740 in order that excess mixture can be readily drained into chamber 750.

In this seventh embodiment, a capillary pathway is provided for isolating a portion of the mixture initially formed in mixing chamber 740. Channel 761 along the front face of block 790 leads to a capillary space 762 to which a reagent can be applied before placing cover plate 795 over base 790. The capillary path proceeds to aperture 763, which traverses block 790. Capillary path 764 continues on the back face of block 790 and terminates at vent space 765 through back plate 795. Actual venting takes place through aperture 767 in back cover plate 797. A solenoid-controlled vent seal (not shown) forms part of the apparatus in which this embodiment is placed for controlling entry and exit of liquids into this capillary pathway. Capillary flow stops at the terminus of channel 764 when channel 764 enters vent space 765, which is non-capillary. The vent seal is then replaced, isolating a predetermined volume of mixture in the space formed by the entire capillary track (761-764). Liquid does not enter channel 724 because of air trapped in this space. After excess mixture is drained from mixing chamber 740, the vent seal (valve) is reopened to allow the isolated mixture to reenter mixing chamber 740. Diluent in chamber 720 further aids to expel trapped liquid in channel 761 and chamber 762 and to draw in trapped liquid from channels 763 nd 764 when valve 722 is opened.

Diluent chambers 710 and 720 are provided with a removable, sealable cover 705 that traps premeasured diluent in these chambers. Cover 705 is removed prior to use in order that flow can occur when valves 712 and 722 are opened. Optional catch spaces 701 and 721 are provided so that chamber 710 and/or 720 can be refilled with diluent, if desired.

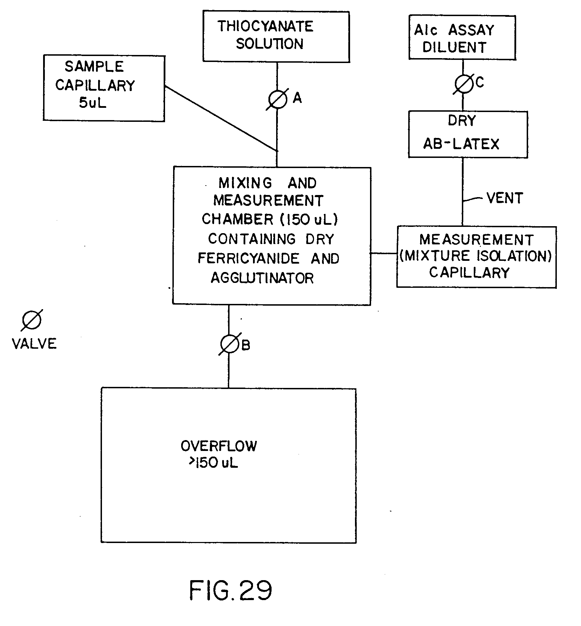

Figure 29 is a schematic diagram showing reagents that would be used with a cartridge of the type as shown in Figure 28 to carry out a specific diagnosis. Hemoglobin Alc, a minor hemoglobin component, is present in normal persons but increases in the presence of hypoglycemia. Hemoglobin Alc measurement therefore provides an assessment of long-term insulin control in diabetics. An analysis requires an initial mixing of whole blood with a first set of reagents to determine total hemoglobin content followed by determination of hemoglobin Alc content on an aliquot of the first mixture. The process steps are shown schematically in Figure 29. A sample from an unmeasured blood drop will be drawn into a sample capillary spontaneously. The sample size is defined by the volume of the sample capillary since flow of blood stops at the junction of the sample capillary and the pathway leading from the denaturant reservoir to the mixing/ reading chamber. When valve A is open, thiocyanate solution will flow toward the mixing chamber, drawing the blood sample with it. The mixture of blood and thiocyanate will fill the mixing chamber, but liquid flow will stop when the mixture reaches an air vent (not shown in Figure 29). Homogeneous mixing of blood and thiocyanate will now occur, driven by a reciprocating mixing plate, and the ferricyanide and agglutinator reagents present in the mixing chamber will dissolve. After about 1 minute, the blood will be lysed and the hemoglobin denatured. At this time, the total hemoglobin will be measured by reading absorbance at 540 nm and 800 nm using a light source and detector that are present in the device into which the cartridge has been inserted. Valve A will then be closed and the vent uncovered to allow a portion of the mixture to flow into the measurement (mixture isolation) capillary. The vent is then closed to prevent the isolated mixture from draining from the mixing chamber during the next step, in which valve B is opened so that all the remaining contents of the reaction chamber drain into the overflow chamber. Once the chamber has drained, valve B will be closed and valve C and the vent opened, allowing diluent to flow through the dry antibody-latex reagent chamber, resuspending the reagent, and displacing the sample of denatured blood (i.e., the isolated mixture) from the measurement capillary into the mixing/reaction chamber. The denatured blood/reagent mixture will then be mixed and assayed for hemoglobin Alc by measurement of the change in turbidity over about 30 seconds. Turbidity increases as a result of agglutination of antibody-coated latex particles, the antibody being specific for hemoglobin Alc.

The location of reagents described in Figure 29 in the apparatus shown in Figures 28A and B is readily apparent. The sample capillary of Figure 29 is measuring chamber 732 of Figure 28B. Thiocyanate solution is present in chamber 710, Alc assay diluent in chamber 720, dry antibody-latex particles in chamber 762, and ferricyanide and agglutinator at different locations in chamber 740. Capillary channels 761-764 provide the measurement (mixture isolation) capillary with vent control occurring at vent chamber 765. Valves A, B, and C of Figure 29 are respectively valves 712, 744, and 722 of Figure 28B. The entire apparatus shown in Figures 28A and B would be approximately 2 inches (5 cm) high and less than 3 inches (7.5 cm) wide with body member 790 being 0.394 inch (1.00 cm) in thickness to provide a standard path length for spectrophotometric analysis of samples in chamber 740.

The invention now being generally described, the following examples are presented for purposes of illustration only. These examples are not intended to be limiting of the invention but can be used to define preferred embodiments.

EXAMPLE 1

An automatic measuring and diluting device was designed and built to exemplify the present invention. This device quantitatively measures a first fluid and automatically adds a specified amount of diluent upon the addition of diluent to a reservoir. The apparatus is shown in Figure 13. The device comprises a solid acrylic block 5 (into which various chambers are formed by drilling and other plastic-cutting operations) along with a flat plate 6 used to form the bottom of the device. A plan view of the device is shown in Figure 13A with lines B-B and C-C showing the locations of the sectional views shown in Figures 13B and 13C. In plan view 13A, the openings of sample chamber 10 and diluent chamber 30 are seen in the top faces of two portions of the block. Measuring chamber 20 is seen at the bottom of chamber 30. Vent 60 is seen on a lower portion of the block, as will be more clearly seen in sectional views.

Figure 13B shows a sectional view in which all internal chambers and channels except for the vent are visible. A capillary channel 12 connects sample chamber 10 to measuring chamber 20, which it enters at junction 14. Since capillary 12 is smaller in diameter than chamber 20, junction 14 is provided with an outward taper as channel 12 enters chamber 20 in order to prevent generation of backpressure due to surface tension forces at this junction. Chamber 20 is a vertical tube having a stop flow junction 50 at the junction of measurement chamber 20 and receiving chamber 40. A stop flow junction 55 is also present at the top of chamber 20 where it connects with diluent chamber 30. Receiving chamber 40 has a lower section 45 that is slightly wider than the upper portion of chamber 40 in order to hold in place a flat rectangular plate (not shown) that is used to mix the fluids in chamber 40. Lower block 6 forms the lower surface of chamber 40 below portion 45. Lower plate 6 is connected to block 5 by screws (not shown).

Figure 13C shows a cross-sectional view through the device at right angles to the view shown in Figure 13B. Vent 60 is seen exiting a lower horizontal surface of block 5 at the opposite end of chamber 40 from stop flow junction 50. The flat plate (not shown) used to mix the liquids in chamber 40 occupies a small portion of the slot shown as section 45 in the lower portion of chamber 40 and mixes fluid by sliding from side to side in the device as shown in Figure 13C. Also visible in Figure 13C is a small section in the roof of receiving chamber 40 at stop flow junction 50 that extends lower into the chamber than the remainder of the roof. This feature is important in preventing liquid from contacting reagent on the upper roof of chamber 40 during the initial stages of filling chamber 40 and also ensures that gas in chamber 40 is displaced by sample and diluent without being trapped as a bubble.

Stop flow junction 50 of the device shown in Figure 13 is the junction of a vertical tube with a horizontal plane that forms an upper surface of chamber 40. The actual dimensions of the interior chambers shown in the embodiment of Figure 13 are set forth in the table below.

Table 1

| Chamber/Tube Number | Radius mm | Length mm | Volume $\mu l$ | Description |
|---|---|---|---|---|
| 10 | 2.35 | 10.77 | 187.0 | Sample Appl. Site |
| 12 | 0.17 | 7.95 | 0.72 | Connecting Channel |
| 20 | 0.52 | 5.87 | 3.1 | Measuring Chamber |
| 30 | 5.00 | 26.21 | 2060.0 | Diluent Chamber |
| 40 | - | 10.00 | 55.0 | Receiving Chamber |

An estimate of the maximum height of the diluent surface over junction 50 was made using the Young-Laplace equation. Assuming R = 0.0515 cm (the radius of the capillary tube 20), $\gamma$ = 60 dynes/cm (a reasonable value for human blood plasma), and $\rho$ (the diluent density) = 1.00 g/cc, a value of 2.4 cm $H_2O$ was obtained. In practice a somewhat different value will be found because of surface defects in the device and contact angle effects.

An equation describing the time to fill capillary tubes 12 and 20 has been developed. The effects of gravity were not allowed for; such effects are not expected to have a major effect in the device of Figure 13.

Time to fill the connecting channel and sample capillary. Sample fluid is applied to application site 10 from which it flows by capillarity into connecting channel 12 and measuring chamber 20. The time to fill these channels is given by:

$$t = \frac{2\pi\mu}{\gamma\cos\theta} \left[ \frac{(L_{12})^2}{R_{12}} + \frac{2L_{20}(L_{12})(R_{20})^3}{(R_{12})^4} + \frac{(L_{20})^2}{R_{20}} \right]$$

where:

R = radius (as in Table 1)

L = length (as in Table 1)

$\mu$ = viscosity: 0.010 g/cm sec

$\gamma$ = surface tension: 60 dynes/cm

$\theta$ = contact angle: 40 degrees

12 = connecting channel

20 = measuring chamber

The calculated fill time was equivalent to the experimentally measured fill time within the expected experimental margin of error.

Receiving chamber 40 is shaped to provide several operating characteristics. It contains a stir bar in lower section 45 that remains flat on the bottom surface of the chamber in order to avoid interferring with the passage of light through the principal long dimension of the chamber. This chamber section has a pathlength of 1cm and flat ends for light to enter and exit, thereby providing a generally useful cuvette segment. The stir bar is made of teflon containing metal particles in order to allow reciprocal motion under the influence of a reciprocating magnetic field. This motion results in mixing of liquids in the chamber.

Two or more reagents can be present in receiving chamber 40. The first reagent can be applied to the surface of chamber 40 near stop flow junction 50 or to the stirrer bar and is contacted by sample and diluent immediately upon the entry of these fluids into chamber 40. A second reagent is applied to the upper horizontal surface of chamber 40 at the left end of the chamber (as viewed in Figure 13C) and is not contacted by liquid until fluid filling the chamber reaches this area. Accordingly, sequential mixing of reagents with fluid entering the reaction and analysis chamber can be accomplished with a controlled time span to allow incubation of the first reagent with the sample prior to contact with the second reagent.

Incubation time is provided by controlling the rate of flow of diluent into the receiving chamber. Flow control can be provided by preparing a restriction in the upper neck of measuring chamber 20 or by providing means in chamber 30 for controlling diluent flow. For example, in the embodiment shown in Figure 13, a washer-like device containing a small central hole was placed into firm contact with the bottom perimeter surface of chamber 30. The rate of fluid flow from chamber 30 is then controlled by the diameter of the hole in the washer. The washer or other flow control device has no other effect on the operation of the device since the flow control at junction 50 depends only on fluid height. For example, the amount of time required to fill the receiving chamber for a cartridge with dimensions given in Table 1 without a flow restrictor is about 0.1 second. With a flow restrictor of radius 0.08 mm and length 7.5 mm, the fill time is 65 seconds (using parameter values given previously).

The time to fill the mixing chamber depends on the dimensions of the diluent chamber, measuring chamber and receiving chamber, as well as the viscosity and density of the liquid. The time to fill is given by:

$$t = \frac{8\mu(R_{30})^2}{pg} \quad \frac{V_{40}}{\pi(R_{30})^6} + L_{20} \quad \frac{1}{(R_{20})^4} - \frac{1}{(R_{30})^4} + L_r \quad \frac{1}{(R_r)^4} - \frac{1}{(R_{30})^4}$$

$$\times \ln \frac{L_{30} + L_{20} + L_r}{L_{30} + L_{20} + L_r - \dfrac{V_{40}}{\pi(R_{30})^2}}$$

where
g = gravitational acceleration
μ = fluid viscosity
p = fluid density
R = radius
V = volume
L = length
30 = diluent chamber
20 = measuring chamber
40 = receiving chamber
r = flow restrictor

The calculated and actual fill times for the receiving chamber were in agreement within experimental error.

Initial observations of the test device have proven its ability to measure, dilute, and mix small volumes of liquids in the desired user-friendly manner for which the device was designed. Experiments used aqueous solutions of dye and human blood plasma as samples and buffered saline solution (100 mM sodium phosphate pH 7, containing 0.13 M NaCl) as diluent. Prior to use the device was subjected to plasma etching to reduce the contact angle with aqueous solutions. The observed contact angle varied from 30-70° depending on etching and previous use of the device. When added to the sample application site all the above fluids filled the connecting channel and the measuring chamber. Flow stopped in all cases at junctions 55 and 50. When diluent was gradually added to a depth of greater than 2 cm in the diluent chamber, sample was rapidly displaced from the measuring chamber above 14 and from the connecting channel 12 back into sample site 10. Flow subsequently (within a few seconds) occurred from the part of measuring channel below 14 into 40. As judged by removal of dye, all the sample in tube 20 below 14 was displaced into 40. Chamber 40 filled without any air being trapped, and flow stopped when vent 60 had filled. Chamber 40 filled from right to left as shown in Figure 13°C, rather than from bottom to top.

EXAMPLE 2

An additional embodiment of the type generally shown previously in Figure 9 was also constructed to demonstrate operation of the invention. The device was constructed from plastic strips 0.65 mm thick which were plasma etched to promote fluid flow. The strips were cut to appropriate sizes, and holes were drilled to serve as various chambers, vents, and application sites. The strips were stuck together with double-stick tape 0.09 mm thick having track designs excised so that capillary tracks were formed between the various cartridge components. Assembly of the plastic strips and double-stick tape to form an actual device is shown in Figure 14. Figure 14A is a plan view while Figure 14B is a sectional view along line D-D shown in Figure 14A. Sample/diluent application site 10/30 was created by drilling a 4.0 mm diameter hole through top plastic strip 5 and middle plastic strip 7. No holes were drilled in bottom plastic strip 9. Chamber 20 was formed by a track design excised from lower double-stick tape 8. The track had a width of 1.0 mm. A similar track 95 joined application site 20/30 to sump 90 which contained stacked filter paper 100 to absorb excess sample. Receiving chamber 40 was formed by drilling a 4.0 mm diameter hole in plastic strip 7. A 0.5 mm diameter hole in plastic strip 5 above chamber 40 provided vent 60. A small steel stir bar 2.5 mm in length (80) was provided in chamber 40. Capillary tracks 20 and 95 were 10 and 5 mm in length, respectively.

One drop of blood was applied to the application site and flowed down both tracks. Flow down track 20 terminated at stop flow junction 50, which was much deeper and wider than the track. All the remaining blood moved down track 95 until application site 10/30 was empty, the blood being absorbed into filter paper 100 in sump 90. In this way, a defined sample volume was isolated in measuring chamber 20. Excess diluent (isotonic saline) was then added to application site 10/30, and stir bar 80 was turned using an external rotating magnetic field (provided by a laboratory stirrer). Contact of stir bar 80 with the leading edge of the blood sample broke the liquid surface and caused flow to resume, flow being driven by gravity. The blood in measuring chamber 20 was completely displaced by diluent, and flow continued until receiving (mixing) chamber 40 was filled with blood and diluent.

EXAMPLE 3

A third embodiment similar to that shown in Figures 7 and 8 was prepared for diluting plasma after plasma was passed through a filter as described in U.S. Application Serial No. 924,633, filed October 29, 1986. This device is shown in Figure 15 in which Figure 15A is a plan view and Figure 15B is a cross-sectional view taken along line E-E of Figure 15A.

The device was prepared from plastic strips and double-stick tape as described for the embodiment of Example 2. The diameters of the various drilled chambers and vents were as follows: sample application site 10, 4.0 mm; diluent appliction site 30, 4.0 mm; receiving (mixing) chamber 40, 4.0 mm; and vent 60, 0.5 mm. Measuring chamber 20 had a length of 2.5 cm and a width of 1.0 mm. Introductory sample channel 12 had a length of 4.0 mm, a width of 1.0 mm, and was located at the extreme left end of track 20 as shown in Figure 15A. A glass fiber filter 0.65 mm thick (Tojo GA200) was present in sample application site 10. Stir bar 80 was identical to that used in the embodiment of Example 2.

One drop of blood was applied to the filter in sample application site 10. Plasma free of red cells emerged into track 12 and flowed into measuring chamber 20 until the measuring chamber was completely filled. Flow stopped at stop flow junctions 50 and 55. Diluent (water) was added to diluent application site 30. Flow did not start until stir bar 80 was rotated. Once flow began, the diluent displaced all the plasma from measuring chamber 20 into receiving (mixing) chamber 40. Flow continued until chamber 40 was full.

To visualize the plasma, dye was added to a blood sample prior to an experiment. There was no significant backflow of sample into track 12 (presumably because the filter provided significant resistance to flow).

All publications and patent applications mentioned in this specification are indicative of the level of skill of those skilled in the art to which this invention pertains.

The invention now being fully described, it will be apparent to one of ordinary skill in the art that many changes and modifications can be made thereto.

**Claims**

1. An apparatus for diluting a liquid sample with a diluent comprising:

    a fixed volume measuring chamber (20)

    a sample application site (10) in liquid donating relationship to said measuring chamber, whereby the sample is delivered from the sample application site to the measuring chamber by capillary or gravitational forces or a combination thereof;

    a receiving chamber (40) in liquid receiving relationship to said measuring chamber;

    a gas vent (60) in said receiving chamber;

    a permanently open stop flow junction (50) between said measuring chamber and said receiving chamber, said stop flow junction having a configuration adapted to provide back pressure caused by surface tension so that the sample will not flow through said stop flow junction at the time when sample is applied to said sample application site

    a diluent application site (30) in liquid donating relationship to the stop flow junction and thereafter the receiving chamber, diluent being delivered from said diluent application site to said receiving chamber by capillary or gravitational forces or a combination thereof.

2. Apparatus according to claim 1, wherein there is a vertical height difference between the diluent application site and the stop flow junction sufficient to provide a hydrostatic pressure capable of overcoming the back pressure when diluent is applied to the diluent application site.

3. Apparatus according to claim 2, wherein back pressure at the stop flow junction when a sample is present at said stop flow junction is from 0.1 to 10cm $H_2O$ and the vertical height difference between the minimum diluent and the stop flow junction is greater than the numerical value in cm of said back pressure.

4. Apparatus according to claim 1, wherein said stop flow junction comprises a change in liquid contact surfaces from a surface having a low contact angle to a surface having a high contact angle.

5. Apparatus according to claim 1, wherein there is a movable part of the apparatus capable of being actuated to contact the liquid surface present at said stop flow junction in order to overcome the back pressure.

6. Apparatus according to claim 5, wherein the stop flow junction comprises an increase in flow path cross-section from a capillary-flow region to a non-capillary-flow region.

7. Apparatus according to claim 1, wherein back pressure at said stop flow junction is greater than hydrostatic pressure available from said sample application site or said diluent application site when sample and diluent, respectively, are both present.

8. Apparatus according to claim 1, wherein the diluent application site has a minimum volume equal to the volume of the receiving chamber and a maximum volume of 10ml.

9. Apparatus according to claim 1, further comprising a mixture isolating chamber hydrostatically connected to said receiving chamber; and

    first valve means selectively preventing flow between said receiving chamber and said isolating chamber, whereby a measured representative sample of the contents of the mixing chamber can be selectively isolated and preserved in the mixture isolating chamber.

10. Apparatus according to claim 9, further comprising:

    second valve means selectively preventing flow from said diluent application site to said receiving chamber.

11. Apparatus according to claim 10, wherein said mixture isolating chamber is in liquid donating relationship to a second receiving chamber.

12. A method of using an apparatus as defined in claim 1 for diluting a liquid sample with a diluent, comprising the steps of:

EP 0 305 210 B1

adding said sample to the sample application site (10)

adding a diluent to the diluent application site (30); and

starting flow at the stop flow junction (50), wherein said diluent transports said sample from the measuring chamber (20) into the receiving chamber (40) without the application of external energy other than gravitational energy or capillary pressure or a combination thereof.

13. A method according to claim 12, wherein starting flow is accomplished by the increase in hydrostatic pressure when said diluent is added.

14. A method according to claim 12, wherein starting flow is accomplished by moving the apparatus or a part of it.

15. A method according to claim 12, wherein starting flow is accomplished by a movable part of said apparatus contacting the sample surface.

16. A method according to claim 12 further comprising:

opening a first valve means selectively controlling passage of the mixture of sample and diluent from said receiving chamber to a hydrostatically connected mixing isolating chamber, wherein a hydrostatically determined portion of said first mixture enters said mixture isolating chamber;

closing said first valve means, thereby isolating said portion from the remainder of said mixture;

transferring said portion to a mixing chamber for diluting with a predetermined volume of a second diluent to form a second mixture.

17. A method according to claim 16, wherein the valve means are opened and closed by a programmed device into which a unitary cartridge comprising said chambers and valve means fits.

18. A method according to claim 17, wherein said programmed device selects a program by which to operate said valve means by detecting a signal from said cartridge when said cartridge is inserted into said programmed device.

**Patentansprüche**

1. Vorrichtung zum Verdünnen einer flüssigen Probe mit einem Verdünner, umfassend:

eine Meßkammer (20) mit vorherbestimmtem Volumen,

eine Probenaufbringstelle (10) in flüssigkeitsabgebender Beziehung zur genannten Meßkammer, wodurch die Probe durch Kapillarwirkung oder Schwerkraft oder eine Kombination davon von der Probenaufbringstelle in die Meßkammer abgegeben wird;

eine Aufnahmekammer (40) in flüssigkeitsaufnehmender Beziehung zur genannten Meßkammer;

eine Gasentlüftungsöffnung (60) in der genannten Aufnahmekammer;

eine permanent geöffnete Stop-Fluß-Verbindung (50) zwischen der genannten Meßkammer und der genannten Aufnahmekammer, wobei die genannte Stop-Fluß-Verbindung eine Konfiguration aufweist, die so ausgebildet ist, daß durch Oberflächenspannung verursachter Gegendruck erzeugt wird, sodaß zur Zeit, wo eine Probe auf die genannte Probenaufbringstelle aufgebracht wird, die Probe nicht durch die genannte Stop-Fluß-Verbindung fließt;

eine Verdünneraufbringstelle (30) in flüssigkeitsabgebender Beziehung zur genannten Stop-Fluß-Verbindung und danach zur Aufnahmekammer, wobei durch Kapillarwirkung oder Schwerkraft oder eine Kombination davon Verdünner von der genannten Verdünneraufbringstelle in die genannte Aufnahmekammer abgegeben wird.

2. Vorrichtung nach Anspruch 1, worin es eine vertikale Höhendifferenz zwischen der Verdünneraufbringstelle und der Stop-Fluß-Verbindung gibt, die ausreicht, um einen hydrostatischen Druck zu erzeugen, der fähig ist, den Gegendruck zu überwinden, wenn Verdünner auf die Verdünneraufbringstelle aufgebracht wird.

3. Vorrichtung nach Anspruch 2, worin der Gegendruck an der Stop-Fluß-Verbindung, wenn eine Probe an der genannten Stop-Fluß-Verbindungvorhanden ist, von 0,1 bis 10cm $H_2O$ beträgt und die vertikale Höhendifferenz zwischen dem Minimum an Verdünner und der Stop-Fluß-Verbindung größer ist als der numerische Wert des genannten Gegendrucks in cm.

26

**4.** Vorrichtung nach Anspruch 1, worin die genannte Stop-Fluß-Verbindung eine Änderung der Flüssigkeitskontaktflächen von einer Fläche mit einem geringen Berührungswinkel zu einer Fläche mit einem großen Berührungswinkel umfaßt.

**5.** Vorrichtung nach Anspruch 1, worin es einen beweglichen Teil der Vorrichtung gibt, der betätigt werden kann, um die an der genannten Stop-Fluß-Verbindung vorhandene Flüssigkeitsoberfläche zu berühren, um den Gegendruck zu überwinden.

**6.** Vorrichtung nach Anspruch 5, worin die Stop-Fluß-Verbindung eine Zunahme des Fließwegquerschnitts von einem Kapillarflußbereich zu einem Nicht-Kapillarflußbereich umfaßt.

**7.** Vorrichtung nach Anspruch 1, worin der Gegendruck an der genannten Stop-Fluß-Verbindung größer als der hydrostatische Druck ist, der von der genannten Probenaufbringstelle oder der genannten Verdünneraufbringstelle verfügbar ist, wenn sowohl Probe als auch Verdünner vorhanden sind.

**8.** Vorrichtung nach Anspruch 1, worin die Verdünneraufbringstelle ein Minimalvolumen, das gleich dem Volumen der Aufnahmekammer ist, und ein Maximalvolumen von 10 ml aufweist.

**9.** Vorrichtung nach Anspruch 1, die weiters eine Mischungsabtrennkammer umfaßt, die hydrostatisch mit der genannten Aufnahmekammer verbunden ist; und
erste Ventileinrichtungen, die den Fluß zwischen der genannten Aufnahmekammer und der genannten Abtrennkammer selektiv verhindern, wodurch eine gemessene repräsentative Probe des Inhalts der Mischkammer selektiv abgetrennt und in der Mischungsabtrennkammer gehalten werden kann.

**10.** Vorrichtung nach Anspruch 9, die weiters umfaßt:
zweite Ventileinrichtungen, die selektiv den Fluß von der genannten Verdünneraufbringstelle zur genannten Aufnahmekammer verhindern.

**11.** Vorrichtung nach Anspruch 10, worin die genannte Mischungsabtrennkammer sich in flüssigkeitsabgebender Beziehung zu einer zweiten Aufnahmekammer befindet.

**12.** Verfahren zur Verwendung einer Vorrichtung nach Anspruch 1 zum Verdünnen einer flüssigen Probe mit einem Verdünner, folgende Schritte umfassend:
das Beschicken der Probenaufbringstelle (10) mit der genannten Probe,
das Beschicken der Verdünneraufbringstelle (30) mit einem Verdünner;
das Ingangsetzen des Flusses an der Stop-Fluß-Verbindung (50), worin der genannte Verdünner die genannte Probe ohne Anwenden anderer äußerer Kräfte als Gravitationsenergie oder Kapillardruck oder einer Kombination davon von der Meßkammer (20) in die Aufnahmekammer (40) transportiert.

**13.** Verfahren nach Anspruch 12, worin das Ingangsetzen des Flusses durch die Erhöhung des hydrostatischen Drucks, wenn der genannte Verdünner hinzugefügt wird, herbeigeführt wird.

**14.** Verfahren nach Anspruch 12, worin das Ingangsetzen des Flusses durch das Bewegen der Vorrichtung oder eines Teils davon herbeigeführt wird.

**15.** Verfahren nach Anspruch 12, worin das Ingangsetzen des Flusses durch einen beweglichen Teil der genannten Vorrichtung, der die Probenoberfläche berührt, herbeigeführt wird

**16.** Verfahren nach Anspruch 12, das weiters umfaßt:
das Öffnen einer ersten Ventileinrichtung, die selektiv den Fluß einer Mischung aus Probe und Verdünner von der genannten Aufnahmekammer in eine hydrostatisch verbundene Mischungsabtrennkammer steuert, wobei eine hydrostatisch bestimmte Teilmenge der genannten ersten Mischung in die genannte Mischungsabtrennkammer eintritt;
das Schließen der genannten ersten Ventileinrichtung, wodurch die genannte Teilmenge vom Rest der genannten Mischung abgetrennt wird;
das Überführen der genannten Teilmenge in eine Mischkammer zum Verdünnen mit einem vorherbestimmten Volumen eines zweiten Verdünners, um eine zweite Mischung zu bilden.

**17.** Verfahren nach Anspruch 16, worin die Ventileinrichtungen durch eine programmierte Vorrichtung geöffnet und geschlossen werden, in die eine Einheitspatrone einsetzbar ist, die die genannten Kammern und Ventileinrichtungen umfaßt.

**18.** Verfahren nach Anspruch 17, worin die genannte programmierte Vorrichtung ein Programm auswählt, durch das die genannten Ventileinrichtungen betrieben werden, indem ein Signal von der genannten Patrone festgestellt wird, wenn die genannte Patrone in die genannte programmierte Vorrichtung eingesetzt wird.

**Revendications**

**1.** Appareil pour diluer un échantillon liquide avec un diluant comprenant :
une chambre (20) de mesure d'un volume fixe
un site (10) d'application d'échantillon dans un liquide donnant une relation à ladite chambre de mesure, ce par quoi l'échantillon est livré du site d'application à la chambre de mesure par des forces capillarité ou de gravitation ou une combinaison de celles-ci;
une chambre (40) de réception dans un liquide recevant une relation de ladite chambre de mesure;
une purge de gaz (60) dans ladite chambre de réception ;
une jonction (50) d'arrêt d'écoulement ouverte en permanence entre ladite chambre de mesure et ladite chambre de réception, ladite jonction d'arrêt d'écoulement ayant une configuration adaptée pour fours une pression en retour causée par la tension de surface de façon que l'échantillon ne s'écoulera pas à travers ladite jonction d'arrêt d'écoulement au moment où l'échantillon est appliqué audit site d'application d'échantillon ;
un site (30) d'application de diluant dans un liquide donnant une relation à la jonction d'arrêt d'écoulement et ensuite à la chambre de réception, le diluant étant livré à partir dudit site d'application de diluant à ladite chambre de réception par des forces de capillarité ou de gravitation ou une combinaison de celles-ci.

**2.** Appareil selon la revendication 1, dans lequel il y a une différence de hauteur verticale entre le site d'application de diluant et la jonction d'arrêt d'écoulement suffisante pour fournir une pression hydrostatique capable de surmonter la pression en retour quand le diluant est appliqué au site d'application de diluant.

**3.** Appareil selon la revendication 2, dans lequel la pression en retour sur la jonction d'arrêt d'écoulement quand un échantillon est présent sur ladite jonction d'arrêt d'écoulement est de 0,1 à 10cm de $H_2O$ et la différence de hauteur verticale entre le diluant minimum et la jonction d'arrêt d'écoulement est supérieure à la valeur numérique en cm de ladite pression en retour.

**4.** Appareil selon la revendication 1, dans lequel ladite jonction d'arrêt d'écoulement comprend un changement dans les surfaces de contact de liquide à partir d'une surface ayant un angle de contact faible à une surface ayant un angle de contact élevé.

**5.** Appareil selon la revendication 1, dans lequel il y a une partie mobile de l'appareil capable d'être actionnée au contact de la surface liquide présente à ladite jonction d'arrêt d'écoulement de façon à surmonter la pression en retour.

**6.** Appareil selon la revendication 5, dans lequel la jonction d'arrêt d'écoulement comprend une augmentation dans la section transversale de la voie d'écoulement à partir d'une région d'écoulement par capillarité à une région d'écoulement capillaire.

**7.** Appareil selon la revendication 1, dans lequel la pression en retour à ladite jonction d'arrêt d'écoulement est supérieure à la pression hydrostatique disponible à partir dudit site d'application d'échantillon ou dudit site d'application du diluant quand l'échantillon et le diluant, respectivement, sont présents ensemble.

**8.** Appareil selon la revendication 1, dans lequel le site d'application de diluant a un volume minimum égal au volume de la chambre de réception et un volume maximum de 10ml.

28

**9.** Appareil selon la revendication 1, comprenant de plus une chambre d'isolement du mélange hydrostatiquement connectée à ladite chambre de réception; et

un moyen de première valve empêchant sélectivement l'écoulement entre ladite chambre de réception et ladite chambre d'isolement, ce par quoi un échantillon représentatif mesuré des contenus de la chambre de mélange peuvent être sélectivement isolés et préservés dans la chambre d'isolement du mélange.

**10.** Appareil selon la revendication 9, comprenant de plus :

un moyen de seconde valve empêchant sélectivement l'écoulement dudit site d'application du diluant à ladite chambre de réception.

**11.** Appareil selon la revendication 10, dans lequel ladite chambre d'isolement du mélange est en relation donnante de liquide à une seconde chambre de réception.

**12.** Procédé d'utilisation d'un appareil tel que défini dans la revendication 1 pour diluer un échantillon liquide avec un diluant, comprenant les étapes de :

ajouter ledit échantillon audit site (10) d'application d'échantillon;

ajouter un diluant au site (30) d'application du diluant et

démarrer l'écoulement à la jonction (50) d'arrêt d'écoulement, dans laquelle ledit diluant transporte ledit échantillon de la chambre de mesure (20) dans la chambre de réception (40) sans l'application d'une énergie externe autre qu'une énergie de gravitation ou qu'une pression capillaire ou qu'une combinaison de celles-ci.

**13.** Procédé selon la revendication 12, dans lequel le démarrage de l'écoulement est accompli par l'augmentation de la pression hydrostatique quand ledit diluant est ajouté.

**14.** Procédé selon la revendication 12, dans lequel le démarrage de l'écoulement est accompli en bougeant l'appareil ou une partie de celui-ci.

**15.** Procédé selon la revendication 12, dans lequel le démarrage de l'écoulement est accompli par une partie mobile dudit appareil contactant la surface de l'échantillon.

**16.** Procédé selon la revendication 12, comprenant de plus :

l'ouverture d'un moyen de première valve contrôlant sélectivement le passage du mélange d'échantillon et du diluant de ladite chambre de réception vers une chambre d'isolement de mélange connectée hydrostatiquement, dans laquelle une partie déterminée hydrostatiquement dudit premier mélange entre dans ladite chambre d'isolement du mélange ;

la fermeture dudit moyen de première valve, isolant par là ladite partie du reste dudit mélange ;

le transfert de ladite partie à une chambre de mélange pour diluer avec un volume prédéterminé d'un second diluant pour former un second mélange.

**17.** Procédé selon la revendication 16, dans lequel les moyens de valve sont ouverts ou fermés par un dispositif programmé dans lequel une cartouche unitaire comprenant lesdites chambres et lesdits moyens de valve s'adapte.

**18.** Procédé selon la revendication 17, dans lequel ledit dispositif programmé sélectionne un programme pour mettre en fonctionnement lesdits moyens de valve par détection d'un signal provenant de ladite cartouche quand ladite cartouche est insérée dans ledit dispositif programmé.

FIG.1

FIG.2

FIG.3

FIG.4

FIG.5

FIG.6

FIG.7

FIG.8

FIG.9

FIG.IOA

FIG.IOB

FIG.II

FIG.I2

EP 0 305 210 B1

FIG.13A

FIG.13B

FIG.13C

FIG.14A

FIG.15A

FIG.14B

FIG.15B

FIG.16A

FIG.16B

32

FIG.17A

FIG.17B

FIG.18

FIG.19

FIG.20

FIG.21

EP 0 305 210 B1

FIG.22

FIG.23

FIG.24

FIG.25

FIG.26

FIG.27

FIG. 28A

FIG. 28B

EP 0 305 210 B1

36

THIOCYANATE
SOLUTION

Alc ASSAY
DILUENT

SAMPLE
CAPILLARY
5uL

⌀ A

⌀ C

DRY
AB-LATEX

MIXING AND
MEASUREMENT
CHAMBER (I5O uL)
CONTAINING DRY
FERRICYANIDE AND
AGGLUTINATOR

— VENT

MEASUREMENT
(MIXTURE ISOLATION)
CAPILLARY

⌀
VALVE

⌀ B

OVERFLOW
>I5O uL

FIG.29